# EUROPEAN PATENT APPLICATION

(11) **EP 4 806 921 A1**
(43) Date of publication of application: **16.09.2026**
(21) Application number: 24889015.4
(22) Date of filing: 29.10.2024
(51) Int. Cl.: C07K 16/30, A61K 47/68, A61P 35/00, A61K 39/00

(54) **ANTI-CEACAM5 ANTIBODY AND USE THEREOF**

(30) Priority: 09.11.2023 KR 20230154645; 10.10.2024 KR 20240137675
(71) Applicant: Daan Biotherapeutics, Inc., Seoul 04323 (KR)
(72) Inventor: CHO, Byoung Chul, Seoul 04425 (KR); PARK, Sae Young, Incheon 21408 (KR); YOON, Ju Gyeong, Seoul 04368 (KR); PARK, Ji Young, Suwon-si Gyeonggi-do 16628 (KR); KIM, Young Seob, Hwaseong-si Gyeonggi-do 18423 (KR); KIM, Dong Ho, Hanam-si Gyeonggi-do 12920 (KR)
(74) Representative: Dragsted Partners A/S
(86) International application number: PCT/KR2024/016648
(87) International publication number: WO 2025/100827

(57) **Abstract**

The present specification relates to an anti-CEACAM5 antibody or binding fragment thereof that specifically binds to CEACAM5 remaining on the surface of cells without binding to sCEACAM5 present in the form of soluble CEACAM5 (sCEACAM5) in blood. Therefore, the antibody of the present invention can be delivered in an increased amount to solid tumor tissues, thus being effective as a therapeutic agent targeting cancer cells expressing CEACAM5.

## Description

### Technical Field

The present disclosure relates to anti-CEACAM5 antibodies and uses thereof.

### Background Art

A carcinoembryonic antigen (CEA) is a glycoprotein associated with cell adhesion. The CEA family belongs to the immunoglobulin superfamily. The CEA family, consisting of 18 genes, is subdivided into two protein subgroups: a CEA-related cell adhesion protein (CEACAM) subgroup and a pregnancy-specific glycoprotein subgroup (Kammerer & Zimmermann, BMC Biology 2010, 8 : 12).

The extracellular domains of CEACAM family members consist of repeated immunoglobulin-like (Ig-like) domains, which are classified into three types of A, B, and N according to sequence homology. CEACAM5 contains seven these domains, that is, N, A1, B1, A2, B2, A3, and B3. The CEACAM is involved in various cellular functions, and plays an important role in regulating cell growth and differentiation through signaling based on intercellular adhesion functions, insulin homeostasis, angiogenesis, and immune regulation. The members of the CEACAM family are involved in various pathophysiological roles, including a role as receptors for microbial pathogens. These members play an important role in carcinogenesis, particularly in cancer detection, progression, and metastasis.

Meanwhile, CEACAM5 (referred to as CEA, also known as CD66e) is a glycoprotein with a molecular weight of approximately 180 kDa. The CEACAM5, which contains seven domains, is anchored to the cell membrane via glycosyl-phosphatidyl-inositol (GPI). The seven domains include a single N-terminal Ig variable domain, and six domains A1-B1-A2-B2-A3-B3 homologous to an Ig constant domain. The CEACAM5 was initially considered as a protein expressed in fetal tissues, but has recently been identified in a plurality of normal adult tissues. In addition, overexpression of CEACAM5 has been observed in a plurality of types of cancer, and additional studies have shown that the overexpression of CEACAM5 is associated with many malignant tumors, often with poor prognosis.

In addition, it has been found that the CEACAM5 was overexpressed in various malignant tumors, such as breast, pancreatic, ovarian, colon, lung, and gastric tumors, and was associated with tumor invasion and metastasis. Accordingly, the importance of CEACAM5 is highlighted as a tumor-associated antigen useful for targeted therapy.

The background art of the disclosure has been prepared to more facilitate understanding of the present disclosure. It should not be understood that the matters described in the background art of the disclosure exist as prior art.

### Detailed Description of the Invention

### Technical Problem

Meanwhile, although progress has been made in the field of immunotherapy, there is still a continuing need for new therapies that are more effective and longer lasted, contain novel targets, and may act as single agents or in combination with known therapies to ultimately generate long-lasting responses in cancer patients.

There remains an unmet need to provide humanized antibodies that recognize specific tumor-specific proteins that are safer, have greater efficacy, and may be used diagnostically and therapeutically in diseases involving tumor-specific protein expression or activation.

Meanwhile, carcinoembryonic antigen-related cell adhesion molecule 5 (CEACAM5) is preferentially overexpressed in some solid cancers, such as colon cancer, pancreatic cancer, lung cancer, stomach cancer, hepatocellular carcinoma, breast cancer, and thyroid cancer, and is highly expressed particularly on the surface of colon, stomach, lung, and uterine tumor cells.

Therefore, the present inventors focused on CEACAM5 to solve the above-mentioned limitations. More specifically, CEACAM5 is a member of the carcinoembryonic antigen (CEA) gene family, belongs to the immunoglobulin (Ig) superfamily, and is also known as cluster of differentiation 66e (CD66e).

In addition, a significant amount of CEACAM5 expressed on the cell surface is detached from the cell surface and exists in the blood in the form of soluble CEACAM5 (sCEACAM5), and the concentration of sCEACAM5 in the serum increases rapidly in cancer patients, reaching a maximum of 2 µg/ml. Antibody therapeutics for anti-CEACAM5 have been studied steadily for a long time, but sCEACAM5 present in the blood is considered as a factor that neutralizes the administered antibodies to reduce the amount of antibody delivered to solid cancer tumor tissue, and consequently inhibits the clinical effect of anti-CEACAM5 antibody therapeutics.

Therefore, the present inventors have confirmed the possibility of a therapeutic agent targeting cancer cells expressing CEACAM5 by developing an antibody that specifically binds to an epitope on CEACAM5 remaining on the cell surface without being detached from the cell surface during antibody screening.

More specifically, the present inventors confirmed the potential of a therapeutic agent targeting cancer cells expressing CEACAM5 by using an antibody that specifically binds to an epitope on CEACAM5 remaining on the cell surface and increasing the amount of an antibody that was delivered to solid cancer tumor tissue by specifically binding to an epitope on CEACAM5 remaining on the cell surface without substantially binding to sCEACAM5, which was detached from the cell surface and present in the blood in the form of soluble CEACAM5 (sCEACAM5).

Finally, the present inventors developed an antibody binding to the epitope on CEACAM5 remaining on the cell surface described above without substantially binding to sCEACAM5 existing in the form of soluble CEACAM5 (sCEACAM5).

At this time, the antibody of the present disclosure may also bind to other epitopes in addition to an A3 domain and/or B3 domain of CEACAM5.

The objects of the present disclosure are not limited to the aforementioned objects, and other objects, which are not mentioned above, will be apparent to those skilled in the art from the following description.

### Technical Solution

To solve the above-described problems, there is provided an anti-CEACAM5 antibody or antigen-binding fragment thereof according to an exemplary embodiment of the present disclosure.

According to a feature of the present disclosure, the anti-CEACAM5 antibody or antigen-binding fragment thereof of the present disclosure may be is a humanized antibody, and the antibody of the present disclosure may bind to CEACAM5 A3 and B3 domains represented by SEQ ID NO: 48. However, it should not be understood that the antibody of the present disclosure does not bind to other domains of CEACAM5.

Specifically, the antibody of the present disclosure may specifically bind to an epitope on CEACAM5 remaining on the cell surface without binding to sCEACAM5, which is detached from the cell surface and exists in the blood in the form of soluble CEACAM5 (sCEACAM5), and more particularly, to CEACAM5 A3 and B3 domains represented by SEQ ID NO: 48, which are predicted to be a portion of the membrane protein remaining after CEACAM5 is cleaved, and also, the antibody may bind to other domains of CEACAM5.

Therefore, when the anti-CEACAM5 antibody or antigen-binding fragment thereof of the present disclosure is used, the anti-CEACAM5 antibody or antigen-binding fragment thereof may be used as a therapeutic agent targeting cancer cells expressing CEACAM5 by binding to CEACAM5 that remains on the cell surface without being detached from the cell surface during antibody screening.

Further, in order to solve the problems, there is provided a pharmaceutical composition for preventing or treating cancer, including the anti-CEACAM5 antibody or antigen-binding fragment thereof according to an exemplary embodiment of the present disclosure as an active ingredient. Furthermore, there may be provided an anticancer adjuvant, a composition for inhibiting the proliferation of CEACAM5 expressing tumor cells, an antibody-drug conjugate, and a pharmaceutical composition for preventing or treating cancer including an antibody-drug conjugate as an active ingredient.

Further, in order to solve the problems, there is provided a method for preventing or treating cancer in a subject, including administering to the subject the pharmaceutical composition for preventing or treating cancer including the anti-CEACAM5 antibody or antigen-binding fragment thereof according to an exemplary embodiment of the present disclosure as an active ingredient.

Details of other exemplary embodiments will be included in the detailed description of the disclosure and the accompanying drawings.

### Effects of the Invention

According to the present disclosure, it is possible to increase the amount of antibody delivered to solid cancer tumor tissue by providing an antibody that specifically binds to an epitope on CEACAM5 remaining on the cell surface.

More specifically, according to the present disclosure, it is possible to further improve a clinical effect of an anti-CEACAM5 antibody therapeutic agent by providing an antibody that specifically binds to an epitope on CEACAM5 remaining on the cell surface to increase the amount of antibody delivered to solid cancer tumor tissue without neutralizing the antibody by soluble CEACAM5 (sCEACAM5) present in the blood.

Furthermore, in the case of using the antibody that specifically binds to an epitope on CEACAM5 remaining on the cell surface, since the antibody is not neutralized by soluble CEACAM5 (sCEACAM5) present in the blood, a therapeutic effect using the antibody may be maintained for a longer period of time, thereby causing a greater immune effect.

The effects according to the present disclosure are not limited to the contents exemplified above, and further various effects are included in the present specification.

### Brief Description of Drawings

FIG. 1 is a diagram illustrating results obtained by purifying a CEACAM5 recombinant protein (full length) produced in cells after transfection into CHO-K1 cells according to an exemplary embodiment of the present disclosure.
FIG. 2 is a diagram illustrating a process of immunizing mice with a CEACAM5 recombinant protein (full length) according to an exemplary embodiment of the present disclosure.
FIG. 3 is a diagram illustrating a result of immunizing mice with a CEACAM5 recombinant protein (full length) according to an exemplary embodiment of the present disclosure.
FIG. 4A is a diagram illustrating results of selecting parental clones that bind to a CEACAM5 full length protein.
FIG. 4B is a diagram illustrating results of selecting subclones that bind to a CEACAM5 full length protein.
FIG. 5 is a diagram illustrating binding capacity of antibodies purified from CHO-K1, CHO-K1-CEACAM5 (full), and CHO-K1-CEACAM5 (A3-B3) cells and six single-antibody clones (hybridoma cells).
FIG. 6 is a diagram illustrating results obtained by purifying a CEACAM5 recombinant protein (A3 and B3 domains) produced in cells after transfection into CHO-K1 cells according to an exemplary embodiment of the present disclosure.
FIG. 7 is a diagram illustrating results of immunizing mice with a CEACAM5 recombinant protein (A3 and B3 domains) according to an exemplary embodiment of the present disclosure.
FIG. 8A is a diagram illustrating results of selecting parental clones binding to CEACAM5 A3 and B3 domain proteins.
FIG. 8B is a diagram illustrating results of selecting subclones binding to CEACAM5 A3 and B3 domain proteins.
FIGS. 9A and 9B are diagrams illustrating the binding capacity of CEACAM5 antibodies purified by monoclones according to an exemplary embodiment of the present disclosure to a CEACAM5 full-length protein.
FIGS. 10A and 10B are diagrams illustrating the binding capacity of CEACAM5 antibodies purified by monoclones according to an exemplary embodiment of the present disclosure to an A549 cell line expressing CEACAM5.
FIGS. 11A and 11B are diagrams illustrating blocking effects of a CEACAM5 antibody due to soluble CEACAM5 according to an exemplary embodiment of the present disclosure.
FIG. 12A is a diagram illustrating analysis of the binding affinity of an antibody 81E3A8 produced by performing a humanization process according to an exemplary embodiment of the present disclosure.
FIG. 12B is a diagram illustrating comparing the binding capacity of an antibody 81E3A8 produced by performing a humanization process according to an exemplary embodiment of the present disclosure to a CHO-k1 (CHO-CEACAM5) cell line expressing CEACAM5 and a CHO-k1 (CHO-CEACAM8) cell line expressing CEACAM8.
FIG. 12C is a diagram illustrating the binding capacity of an antibody 81E3A8 produced by performing a humanization process according to an exemplary embodiment of the present disclosure to H3122, KATO III, and MKN45 cell lines expressing CEACAM5.
FIGS. 13A and 13B are diagrams illustrating analysis of the binding affinity of an antibody 45A2E10 produced by performing a humanization process according to an exemplary embodiment of the present disclosure.
FIG. 14 is a diagram illustrating the binding capacity of an antibody 45A2E10 produced by performing a humanization process according to an exemplary embodiment of the present disclosure to a CHO-k1 (CHO-CEACAM5) cell line expressing CEACAM5.
FIG. 15 is a diagram illustrating binding affinity of various clonal antibodies 94E12E2 produced by performing a humanization process according to an exemplary embodiment of the present disclosure confirmed by ELISA assay.
FIG. 16 is a diagram illustrating binding affinity of various clonal antibodies 94E12E2 produced by performing a humanization process according to an exemplary embodiment of the present disclosure confirmed by flow cytometry.
FIGS. 17A and 17B are diagrams illustrating analysis of binding affinity of antibody 36B5H8 clones that are modified with sequences corresponding to post-translational modification (PTM) according to an exemplary embodiment of the present disclosure.

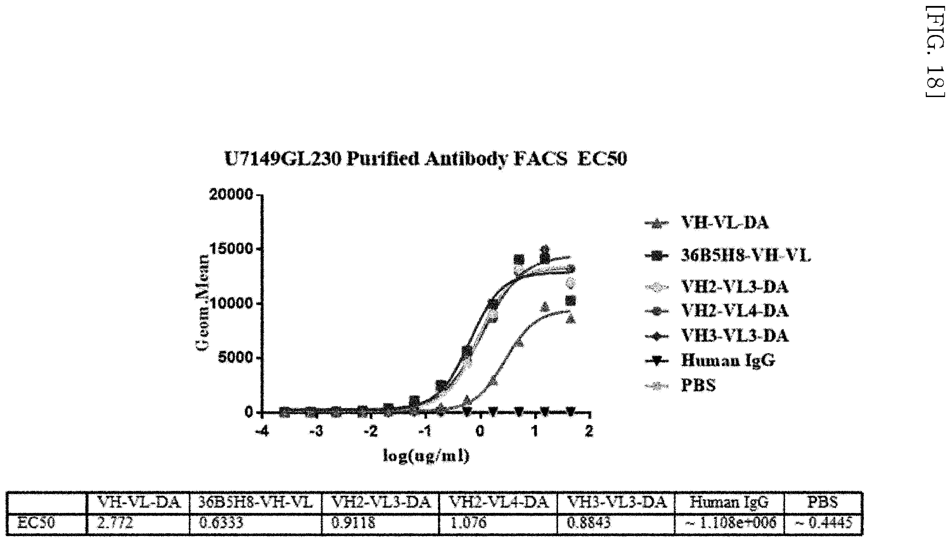

FIG. 18 is a diagram illustrating binding capacity of selected antibodies using CHO-K1-CEACAM5 cells after humanization and post-translational modification (PTM) processes according to an exemplary embodiment of the present disclosure.
FIG. 19A is a diagram illustrating a structure of a CEACAM5 (full length) antigen according to an exemplary embodiment of the present disclosure.
FIG. 19B is a diagram illustrating A3 and B3 domains of a CEACAM5 antigen according to an exemplary embodiment of the present disclosure.

### Modes of the Invention

Advantages and features of the present disclosure, and methods for accomplishing the same will be more clearly understood from exemplary embodiments to be described below in detail with reference to the accompanying drawings. However, the present disclosure is not limited to the exemplary embodiments set forth below, and will be embodied in various different forms. The exemplary embodiments are just for rendering the disclosure of the present disclosure complete and are set forth to provide a complete understanding of the scope of the disclosure to those skilled in the art to which the present disclosure pertains.

The term "antibody" as used in the present disclosure refers to not only conventional antibodies and fragments thereof, but also single domain antibodies and fragments thereof, particularly, variable heavy chains of the single domain antibodies, and chimeric, humanized, bispecific, or multispecific antibodies. The antibodies have five types of IgM, IgD, IgG, IgA, and IgE, and each includes a heavy chain made from heavy chain constant region genes µ, δ, γ, α, and ε. In antibody technology, IgG is mainly used, and there are four isotypes of IgG1, IgG2, IgG3, and IgG4, and the structural and functional characteristics of each may be different. In addition, the IgG forms a very stable Y-shaped structure (molecular weight, about 150 kDa) consisting of only two heavy chain (about 50 kDa) proteins and two light chain (about 25 kDa) proteins. The light and heavy chains of the antibody are divided into variable regions having different amino acid sequences for each antibody and constant regions having the same amino acid sequence. The heavy chain constant region contains CH1, H (hinge), CH2, and CH3 domains. Each domain consists of two β-sheets and is linked by an intramolecular disulfide bond. The two variable regions of the heavy and light chains are combined to form an antigen binding site. There is one antigen binding site on each of two Y-shaped arms, wherein a part capable of binding to the antigen is called Fab (antibody binding fragment), and a part bind that does not bind to the antigen is called Fc (crystalizable fragment). The Fab and Fc are linked by a flexible hinge region.

The term "humanized antibody" as used in the present disclosure refers to an antibody that is wholly or partially non-human in origin and is modified to replace specific amino acids, for example, in framework regions of the VH and VL domains, in order to evade or minimize an immune response in humans. The constant domains of the humanized antibody are, in most cases, human CH and CL domains.

At this time, the term "heavy chain" as used in the present disclosure, when used in relation to the antibody, may refer to any separate type, for example, alpha (α), delta (δ), epsilon (ε), gamma (γ), and mu (µ), based on the amino acid sequence of the constant domain, which generate IgA, IgD, IgE, IgG, and IgM classes of each antibody, including subclasses of IgG, for example, IgG1, IgG2, IgG3, and IgG4.

The term "light chain" as used in the present disclosure, when used in relation to the antibody, may refer to any separate type, for example, kappa (K) or lambda (λ), based on the amino acid sequence of the constant domain. The light chain amino acid sequences are known in the art. In a specific exemplary embodiment, the light chain is a human light chain.

The term "immunoglobulin" as used in the present disclosure refers to an immune molecule derived from any of commonly known isotypes, including IgA, secretory IgA, IgG, and IgM, but is not limited thereto. The IgG subclass is also well known to those skilled in the art, and includes human IgG1, IgG2, IgG3, and IgG4, but is not limited thereto. Many molecules described herein are immunoglobulins. As used herein, the "isotype" refers to an antibody class or subclass (e.g., IgM or IgG1) encoded by heavy chain constant region genes. Furthermore, immunoglobulin is typically a tetrameric molecule consisting of two identical pairs of polypeptide chains, each pair having one "light chain" (about 25 kDa) and one "heavy chain" (about 50 to 70 kDa).

The term "antigen-binding fragment" as used in the present disclosure means a protein that includes a portion of an antibody capable of specifically binding to an antigen while lacking some amino acids compared to a full-length chain of the antibody, and refers to a protein including a portion that binds to an antigen or target protein and, optionally, a scaffold or framework site, such that an antigen binding site adopts a conformation that facilitates binding of the antigen binding molecule to the antigen. Representative examples of the antigen binding molecule may include scFv, human, mouse or rabbit antibodies; humanized antibodies; chimeric antibodies; recombinant antibodies; single-chain antibodies; single-domain antibodies (e.g., VHH); diabodies; triabodies; tetrabodies; Fab, Fab', F(ab')2, and Fv fragments; IgD antibodies; IgE antibodies; IgM antibodies; IgG1 antibodies; IgG2 antibodies; IgG3 antibodies; or IgG4 antibodies and fragments thereof, but are not limited thereto. Such antigen binding molecules may include alternative protein scaffolds or artificial scaffolds, for example, having grafted complementarity determining regions (CDRs) or CDR derivatives. In this case, the scaffolds include not only antibody-derived scaffolds, for example, including mutations introduced to stabilize a three-dimensional structure of the antigen binding molecule, but also completely synthetic scaffolds, for example, including biocompatible polymers, but are not limited thereto. Furthermore, the antigen binding molecule may have at least one binding site. When more than one binding site is present, the binding sites may be identical to or different from each other. That is, the antibody in the present disclosure has antigen binding capacity and may be a single-chain composite polypeptide including amino acid sequences homologous or similar to the variable regions of immunoglobulin light and heavy chains, i.e., a linked VH-VL or single-chain Fv (scFv).

As used in the present disclosure, the term "Fab fragment" is a monovalent fragment having VL, VH, CL and CH domains; "F(ab')2 fragment" is a bivalent fragment having two Fab fragments linked by a disulfide bridge at a hinge region; "Fv fragment" has VH and VL domains of a single arm of an antibody; and "dAb fragment" has an antigen-binding fragment of a VH domain, a VL domain, or a VH or VL domain.

As used in the present disclosure, the terms "single-chain antibody" and "single-chain variable fragment (scFv)" are used interchangeably and refer to an antigen binding molecule in which the VL and VH regions are linked via a linker to form a continuous protein chain, wherein the linker is sufficiently long to allow the protein chain itself to be folded and form a monovalent antigen binding site. More specifically, scFv refers to a fusion of the variable regions of the heavy and light chains of an immunoglobulin, linked together by a single-chain (usually serine, glycine) linker. The single-chain antibody has antigen binding capacity and may be a single-chain composite polypeptide (linked VH-VL or single-chain Fv (scFv)) including amino acid sequences homologous or similar to the variable regions of immunoglobulin light and heavy chains. Both VH and VL may copy a monoclonal antibody sequence, or one or both of the chains may include a CDR-FR construct. Separate polypeptides similar to the variable regions of the light and heavy chains are linked by a polypeptide linker.

The term "CDR" as used in the present disclosure refers to a site binding to an antigen, as a hypervariable region, which is a region having a different amino acid sequence for each antibody in the heavy and light chain variable regions of an antibody. In the conformation of the antibody, the CDR has a loop shape on the surface of the antibody, and has a framework region (FR) that structurally supports under the loop. There are three loop structures in each of the heavy and light chains, and these six loop region structures are combined with each other to come into direct contact with the antigen. More specifically, the framework region may help maintain the appropriate conformation of the CDR to promote binding between the antigen binding molecule and the antigen. There are three CDRs in a variable region of each of the heavy and light chains, which are designated as CDR1, CDR2, and CDR3 for each variable region. The exact boundaries of the CDRs are defined differently depending on a different system.

The term "variable region" or "variable domain" as used in the present disclosure is used interchangeably and refers to a portion of an antibody, usually a portion of the light or heavy chain, typically an amino-terminal end of the antibody, and differs extensively in sequence between antibodies and is used in binding and specificity of a specific antibody to a specific antigen. The sequence variability is concentrated in regions called complementarity determining regions (CDRs), while a more highly conserved region of the variable domain is called a framework region (FR). The CDRs of the light and heavy chains are primarily responsible for the interaction and specificity of the antibody with the antigen.

The term "binding affinity" as used in the present disclosure refers to the total strength of non-covalent interactions between a single binding site of a molecule (e.g., an antigen binding molecule such as an antibody) and its binding partner (e.g., an antigen). Unless otherwise indicated, "binding affinity" as used herein refers to intrinsic binding affinity, which reflects a 1 : 1 interaction between members (e.g., antibody and antigen) of a binding pair. The binding affinity of a molecule X to its partner Y may generally be represented by a dissociation constant (KD). The binding affinity may be measured and/or expressed in many methods known in the art, including an equilibrium dissociation constant (KD) and an equilibrium association constant (KA), but is not limited thereto. The KD is calculated from Equation of koff/kon, while the KA is calculated from Equation of kon/koff. The kon refers to, for example, an association rate constant of an antibody to an antigen, and the koff refers to, for example, a dissociation rate constant of an antibody to an antigen. The kon and koff may be determined by standard techniques known to those skilled in the art, such as BIAcore^{®} or KinExA or surface plasmon resonance.

As used in the present disclosure, the term "substantially not bind" means that the binding domain of the present disclosure does not bind to an antigen or protein other than CEACAM5 present on the cell surface, and a target cell surface antigen, for example, soluble CEACAM5 (sCEACAM5), that is, when CEACAM5 present on the cell surface and an antigen or protein other than CEACAM5, and a target cell surface antigen, for example, soluble CEACAM5 (sCEACAM5) are present at the same time, a blocking effect of the CEACAM5 antibodies due to soluble CEACAM5 is less than about 60%, about 50% or less, about 20% or less, and more preferably about 15% or less.

The term "bispecific" as used in the present disclosure means recognizing and binding to two different antigens or epitopes.

The term "antibody-drug conjugate" as used in the present disclosure refers to an antibody molecule coupled to a therapeutic agent or label, for example, a cytotoxic agent. The antibody molecule may be coupled directly or indirectly, for example, to a non-antibody moiety via a linker. As used in the present disclosure, the binding of the antibody or antigen-binding fragment thereof of the present disclosure to CEACAM5 may mean binding to an epitope on CEACAM5 that remains on the cell surface. For example, the antibody or antigen-binding fragment thereof does not substantially bind or binds at a significantly lower level to an epitope of soluble CEACAM5 (sCEACAM5) that is detached from the cell surface and present in the blood, and in an exemplary embodiment of the present disclosure, binds to the A3 and B3 domains, which are expected to be the remaining portion of the membrane protein after CEACAM5 is cleaved, with high binding capacity, and thus it may be meant that the binding affinity of the antibody or antigen-binding fragment thereof of the present disclosure to CEACAM5 located on the tumor cell surface is not substantially inhibited even when soluble CEACAM5 (sCEACAM5) is present together. In addition, without limitation, the antibody of the present disclosure may also bind to other epitopes in addition to the A3 and B3 domains of CEACAM5.

The term "conservative amino acid substitution" as used in the present disclosure means replacing an amino acid residue with an amino acid residue having a similar side chain. Families of amino acid residues having side chains are defined in the art. These families include amino acids having basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). In a specific exemplary embodiment, one or more amino acid residues within CDR(s) or framework region(s) of the antibody or antigen binding molecule (or fragment thereof) provided herein may be replaced with amino acid residues having a similar side chain.

The terms "constant region" and "constant domain" as used in the present disclosure are interchangeable and have a common meaning in the art. The constant region is an antibody moiety, e.g., a carboxyl terminal portion of the light chain and/or heavy chain, that is not directly involved in binding of the antibody to the antigen, but may exhibit various effector functions, such as interactions with an Fc receptor. The constant region of an immunoglobulin molecule generally has a more conserved amino acid sequence than the immunoglobulin variable domain.

The term "epitope" as used in the present disclosure may mean a region of a specific antigen molecule capable of specifically binding to a specific antibody. The epitope may be, for example, contiguous amino acids of a polypeptide (linear or contiguous epitope), or the epitope may be, for example, gathered together from a polypeptide or two or more non-contiguous regions of polypeptides (conformational, non-linear, discontinuous, or non-contiguous epitope). In a specific exemplary embodiment, the epitope to which the antibody binds may be determined, for example, by NMR spectroscopy, X-ray diffraction crystallography, ELISA assay, hydrogen/deuterium exchange coupled with mass spectrometry (e.g., liquid chromatography electrospray mass spectrometry), array-based oligo-peptide scanning assays, and/or mutagenesis mapping (e.g., site-directed mutagenesis mapping). In the case of X-ray crystallography, crystallization may be accomplished using any of methods known in the art. Furthermore, peptide sequences derived from the epitope may be used alone or in combination with a carrier moiety by applying methods known in the art, to immunize animals, and to produce additional polyclonal or monoclonal antibodies. Isolated peptides derived from the epitope may be used as therapeutic agents in diagnostic methods for detecting antibodies, and when inhibition of such antibodies is required.

In an exemplary embodiment of the present disclosure, the antibody of the present disclosure may specifically bind to CEACAM5 represented by SEQ ID NO: 47, and more specifically, a region of a specific antigen molecule to which the antibody of the present disclosure may specifically bind may be A3 and B3 domains of CEACAM5 including an amino acid sequence represented by SEQ ID NO: 48, but is not limited thereto.
SEQ ID NO: 47 (CEACAM5) 
KLTIESTPFNVAEGKEVLLLVHNLPQHLFGYSWYKGERVDGNRQIIGYVIGTQQA
TPGPAYSGREIIYPNASLLIQNIIQNDTGFYTLHVIKSDLVNEEATGQFRVYPELPKPSISS
NNSKPVEDKDAVAFTCEPETQDATYLWWVNNQSLPVSPRLQLSNGNRTLTLFNVTRNDTASY
KCETQNPVSARRSDSVILNVLYGPDAPTISPLNTSYRSGENLNLSCHAASNPPAQYSWFVNG
TFQQSTQELFIPNITVNNSGSYTCQAHNSDTGLNRTTVTTITVYAEPPKPFITSNNSNPVED
EDAVALTCEPEIQNTTYLWWVNNQSLPVSPRLQLSNDNRTLTLLSVTRNDVGPYECGIQNEL
SVDHSDPVILNVLYGPDDPTISPSYTYYRPGVNLSLSCHAASNPPAQYSWLIDGNIQQHTQE
LFISNITEKNSGLYTCQANNSASGHSRTTVKTITVSAELPKPSISSNNSKPVEDKDAVAFTC
EPEAQNTTYLWWVNGQSLPVSPRLQLSNGNRTLTLFNVTRNDARAYVCGIQNSVSANRSDPV
TLDVLYGPDTPIISPPDSSYLSGANLNLSCHSASNPSPQYSWRINGIPQQHTQVLFIAKITP
NNNGTYACFVSNLATGRNNSIVKSITVSASGTSPGLSAGATVGIMIGVLVGVALI
SEQ ID NO: 48 (A3 and B3 domains of CEACAM5)

VSAELPKPSISSNNSKPVEDKDAVAFTCEPEAQNTTYLWWVNGQSLPVSPRLQLS
NGNRTLTLFNVTRNDARAYVCGIQNSVSANRSDPVTLDVLYGPDTPIISPPDSSYLSGANLN
LSCHSASNPSPQYSWRINGIPQQHTQVLFIAKITPNNNGTYACFVSNLATGRNNSIVKSITV
SASGTSPGLS

In addition, in a specific exemplary embodiment of the present disclosure, an anti-CEACAM5 antibody or antigen-binding fragment thereof that specifically binds to the A3 and B3 domains of CEACAM5 including the amino acid sequence represented by SEQ ID NO: 48 may be (1) a 81E3A8 antibody or antigen-binding fragment thereof including a heavy chain CDR1 including an amino acid sequence represented by SEQ ID NO: 1 or an amino acid sequence having at least 80%, at least 85%, at least 90%, or at least 95% sequence homology thereto, a heavy chain CDR2 including an amino acid sequence represented by SEQ ID NO: 2 or an amino acid sequence having at least 80%, at least 85%, at least 90%, or at least 95% sequence homology thereto, a heavy chain CDR3 including an amino acid sequence represented by SEQ ID NO: 3 or an amino acid sequence having at least 80%, at least 85%, at least 90%, or at least 95% sequence homology thereto, a light chain CDR1 including an amino acid sequence represented by SEQ ID NO: 4 or an amino acid sequence having at least 80%, at least 85%, at least 90%, or at least 95% sequence homology thereto, a light chain CDR2 including an amino acid sequence represented by SEQ ID NO: 5 or an amino acid sequence having at least 80%, at least 85%, at least 90%, or at least 95% sequence homology thereto, and a light chain CDR3 including an amino acid sequence represented by SEQ ID NO: 6 or an amino acid sequence having at least 80%, at least 85%, at least 90%, or at least 95% sequence homology thereto.

In a specific exemplary embodiment of the present disclosure, the anti-CEACAM5 antibody or antigen-binding fragment thereof that specifically binds to the A3 and B3 domains of CEACAM5 including the amino acid sequence represented by SEQ ID NO: 48 may be (2) a 45A2E10 antibody or antigen-binding fragment thereof including a heavy chain CDR1 including an amino acid sequence represented by SEQ ID NO: 13 or an amino acid sequence having at least 80%, at least 85%, at least 90%, or at least 95% sequence homology thereto, a heavy chain CDR2 including an amino acid sequence represented by SEQ ID NO: 14 or an amino acid sequence having at least 80%, at least 85%, at least 90%, or at least 95% sequence homology thereto, a heavy chain CDR3 including an amino acid sequence represented by SEQ ID NO: 15 or an amino acid sequence having at least 80%, at least 85%, at least 90%, or at least 95% sequence homology thereto, a light chain CDR1 including an amino acid sequence represented by SEQ ID NO: 16 or an amino acid sequence having at least 80%, at least 85%, at least 90%, or at least 95% sequence homology thereto, a light chain CDR2 including an amino acid sequence represented by SEQ ID NO: 17 or an amino acid sequence having at least 80%, at least 85%, at least 90%, or at least 95% sequence homology thereto, and a light chain CDR3 including an amino acid sequence represented by SEQ ID NO: 18 or an amino acid sequence having at least 80%, at least 85%, at least 90%, or at least 95% sequence homology thereto.

In a specific exemplary embodiment of the present disclosure, the anti-CEACAM5 antibody or antigen-binding fragment thereof that specifically binds to the A3 and B3 domains of CEACAM5 including the amino acid sequence represented by SEQ ID NO: 48 may be (3) a 94E12E2 antibody or antigen-binding fragment thereof including a heavy chain CDR1 including an amino acid sequence represented by SEQ ID NO: 24 or an amino acid sequence having at least 80%, at least 85%, at least 90%, or at least 95% sequence homology thereto, a heavy chain CDR2 including an amino acid sequence represented by SEQ ID NO: 25 or an amino acid sequence having at least 80%, at least 85%, at least 90%, or at least 95% sequence homology thereto, a heavy chain CDR3 including an amino acid sequence represented by SEQ ID NO: 26 or an amino acid sequence having at least 80%, at least 85%, at least 90%, or at least 95% sequence homology thereto, a light chain CDR1 including an amino acid sequence represented by SEQ ID NO: 27 or an amino acid sequence having at least 80%, at least 85%, at least 90%, or at least 95% sequence homology thereto, a light chain CDR2 including an amino acid sequence represented by SEQ ID NO: 28 or an amino acid sequence having at least 80%, at least 85%, at least 90%, or at least 95% sequence homology thereto, and a light chain CDR3 including an amino acid sequence represented by SEQ ID NO: 29 or an amino acid sequence having at least 80%, at least 85%, at least 90%, or at least 95% sequence homology thereto.

In a specific exemplary embodiment of the present disclosure, the anti-CEACAM5 antibody or antigen-binding fragment thereof that specifically binds to the A3 and B3 domains of CEACAM5 including the amino acid sequence represented by SEQ ID NO: 48 may be (4) a 36B5H8 antibody or antigen-binding fragment thereof including a heavy chain CDR1 including an amino acid sequence represented by SEQ ID NO: 40 or an amino acid sequence having at least 80%, at least 85%, at least 90%, or at least 95% sequence homology thereto, a heavy chain CDR2 including an amino acid sequence represented by SEQ ID NO: 41 or 46 or an amino acid sequence having at least 80%, at least 85%, at least 90%, or at least 95% sequence homology thereto, a heavy chain CDR3 including an amino acid sequence represented by SEQ ID NO: 42 or an amino acid sequence having at least 80%, at least 85%, at least 90%, or at least 95% sequence homology thereto, a light chain CDR1 including an amino acid sequence represented by SEQ ID NO: 43 or an amino acid sequence having at least 80%, at least 85%, at least 90%, or at least 95% sequence homology thereto, a light chain CDR2 including an amino acid sequence represented by SEQ ID NO: 44 or an amino acid sequence having at least 80%, at least 85%, at least 90%, or at least 95% sequence homology thereto, and a light chain CDR3 including an amino acid sequence represented by SEQ ID NO: 45 or an amino acid sequence having at least 80%, at least 85%, at least 90%, or at least 95% sequence homology thereto, but the present disclosure is not limited thereto.

In addition, in another specific exemplary embodiment of the present disclosure, the anti-CEACAM5 antibody or antigen-binding fragment thereof including (1) the heavy chain CDR1 including the amino acid sequence represented by SEQ ID NO: 1, the heavy chain CDR2 including the amino acid sequence represented by SEQ ID NO: 2, the heavy chain CDR3 including the amino acid sequence represented by SEQ ID NO: 3, the light chain CDR1 including the amino acid sequence represented by SEQ ID NO: 4, the light chain CDR2 including the amino acid sequence represented by SEQ ID NO: 5, and the light chain CDR3 including the amino acid sequence represented by SEQ ID NO: 6 may be a humanized antibody including any one of framework sequences represented by SEQ ID NOs: 7 or 11 in the heavy chain region, and any one of framework sequences represented by SEQ ID NOs: 8, 9, 10, and 12 in the light chain region.

In yet another specific exemplary embodiment, the anti-CEACAM5 antibody or antigen-binding fragment thereof including (2) the heavy chain CDR1 including the amino acid sequence represented by SEQ ID NO: 13, the heavy chain CDR2 including the amino acid sequence represented by SEQ ID NO: 14, the heavy chain CDR3 including the amino acid sequence represented by SEQ ID NO: 15, the light chain CDR1 including the amino acid sequence represented by SEQ ID NO: 16, the light chain CDR2 including the amino acid sequence represented by SEQ ID NO: 17, and the light chain CDR3 including the amino acid sequence represented by SEQ ID NO: 18 may be a humanized antibody including any one of framework sequences represented by SEQ ID NO: 19 or 21 in the heavy chain region, and any one of framework sequences represented by SEQ ID NOs: 20, 22, and 23 in the light chain region.

In yet another specific exemplary embodiment, the anti-CEACAM5 antibody or antigen-binding fragment thereof including (3) the heavy chain CDR1 including the amino acid sequence represented by SEQ ID NO: 24, the heavy chain CDR2 including the amino acid sequence represented by SEQ ID NO: 25, the heavy chain CDR3 including the amino acid sequence represented by SEQ ID NO: 26, the light chain CDR1 including the amino acid sequence represented by SEQ ID NO: 27, the light chain CDR2 including the amino acid sequence represented by SEQ ID NO: 28, and the light chain CDR3 including the amino acid sequence represented by SEQ ID NO: 29 may be a humanized antibody including any one of framework sequences represented by SEQ ID NOs: 30, 32, 34, 36 and 38 in the heavy chain region, and any one of framework sequences represented by SEQ ID NOs: 31, 33, 35, 37 and 39 in the light chain region.

In yet another specific exemplary embodiment, in the anti-CEACAM5 antibody or antigen-binding fragment thereof including (4) the heavy chain CDR1 including the amino acid sequence represented by SEQ ID NO: 40, the heavy chain CDR2 including the amino acid sequence represented by SEQ ID NO: 41 or 46, the heavy chain CDR3 including the amino acid sequence represented by SEQ ID NO: 42, the light chain CDR1 including the amino acid sequence represented by SEQ ID NO: 43, the light chain CDR2 including the amino acid sequence represented by SEQ ID NO: 44, and the light chain CDR3 including the amino acid sequence represented by SEQ ID NO: 45, in the case where the heavy chain CDR2 is the heavy chain CDR2 including the amino acid sequence represented by SEQ ID NO: 46, the heavy chain CDR2 including the amino acid sequence represented by SEQ ID NO: 41 may be modified with a sequence corresponding to a post-translational modification (PTM) as the heavy chain CDR2 including the amino acid sequence represented by SEQ ID NO: 46, but is not limited thereto.

As used in the present disclosure, the terms "peptide", "polypeptide", and "protein" are used interchangeably herein and mean a compound consisting of amino acid residues covalently linked by peptide bonds. The polypeptide, protein, or peptide needs to contain at least two amino acids, but there is no limitation to the maximum number of amino acids that may include an amino acid sequence of a protein or peptide. The terms as used herein refer to both a single chain, commonly also referred to as peptide, oligopeptide, and oligomer, and a longer chain, commonly referred to as protein. The "polypeptide" includes particularly, for example, biologically active fragments, substantially homologous polypeptides, oligopeptides, homodimers, heterodimers, variants of polypeptides, modified polypeptides, derivatives, analogs, and fusion proteins. The term "polypeptide" includes natural peptides, recombinant peptides, synthetic peptides, or combinations thereof.

The terms "transduction" and "transduced" as used in the present disclosure refer to a method of introducing foreign DNA into cells via a viral vector. In some exemplary embodiments, the vector is a retroviral vector, a DNA vector, an RNA vector, an adenovirus vector, a baculovirus vector, an Epstein-Barr virus vector, a papovavirus vector, a vaccinia virus vector, a herpes simplex virus vector, an adenovirus-associated vector, a lentivirus vector, or any combination thereof.

As used in the present disclosure, the term "anticancer" includes "prevention" and "treatment", wherein "prevention" means any action that inhibits the proliferation of cancer or delays the progression of cancer by administering the anticancer agent, and "treatment" means any action that improves or beneficially changes the symptoms of cancer by administering the antibody of the present disclosure.

As used in the present disclosure, the term "cancer" may be at least one of pancreatic cancer, melanoma, lung cancer, and myeloma, but is not limited thereto, and cancer or tumors that have CEACAM5 as a receptor and whose immune checkpoint pathway operates abnormally are not particularly limited, and may include solid cancer and blood cancer. However, preferably, the cancer may be lung cancer.

As used in the present disclosure, the "cell therapeutic agent" means a medicine used for the purpose of prevention and treatment through a series of actions that change the biological characteristics of cells by proliferating and selecting living autologous, allogenic, and xenogenic cells in vitro to restore the functions of cells and tissues. More specifically, the cell therapeutic agent may be cytotoxic T cells or natural killer cells.

In addition, in an exemplary embodiment of the present disclosure, a pharmaceutical composition for preventing or treating cancer including the anti-CEACAM5 antibody or binding fragment thereof of the present disclosure as an active ingredient may be used as a cell therapeutic agent including the anti-CEACAM5 antibody or binding fragment thereof as an active ingredient, but is not limited thereto. More specifically, in an exemplary embodiment, the pharmaceutical composition for preventing or treating cancer including the anti-CEACAM5 antibody or binding fragment thereof of the present disclosure as an active ingredient may be used in adaptive cell therapy (ACT). At this time, the adaptive cell therapy (ACT) is a cell therapy method that collects immune cells in the body, strengthens the immune cells, or genetically modifies the immune cells, and introduces the immune cells again. Representatively, a method for strengthening cellular immunity against cancer cells includes cell therapy agents using tumor infiltrating lymphocytes (TIL), T cell receptors (TCR), and chimeric antigen receptors (CAR), and the like.

As used in the present disclosure, the term "subject" may be humans or mammals other than humans, and means a subject whose disease may be alleviated, suppressed, or treated by administering the pharmaceutical composition for preventing or treating cancer including the anti-CEACAM5 antibody or binding fragment thereof of the present disclosure as an active ingredient, or a subject suffering from a disease.

As used in the present disclosure, the term "administration" means introducing an effective amount of material into a subject by an appropriate method, and the route of administration of the composition including the anti-CEACAM5 antibody or antigen-binding fragment thereof of the present disclosure may be administered through a general route that may reach a target tissue. Specifically, the administration may be parenteral administration (for example, intravenous, subcutaneous, intraperitoneal, or local administration) depending on the intended use, and may be preferably intravenous administration. In some cases, local administration may also be preferable for administration to solid tumors to allow rapid and easy access of the antibody. A dose thereof varies in the range thereof according to the weight, age, sex, health condition, diet, administration time, administration method, and excretion rate of a patient, the severity of disease, etc. A single dose may be administered in an amount of about 0.001 to 10 mg/kg in a daily or weekly unit. The effective amount may be adjusted according to the opinion of a physician treating the patient.

As used in the present disclosure, the terms "immunospecifically bind," "immunospecifically recognize," "specifically bind," and "specifically recognize" are similar terms and are used interchangeably with respect to antigen binding molecules, and mean that a given molecule preferentially binds to an antigen (e.g., epitope or immune complex) to nonspecific binding, but do not mean that the given molecule does not bind to any antigen or epitope other than the given antigen or epitope. For example, as used in the present disclosure, "specifically binding to the CEACAM5 A3 and B3 domains" does not mean that the corresponding binding molecule (e.g., antibody) needs to bind only to the CEACAM5 A3 and B3 domains.

### Example 1: Selection of antibodies binding to CEACAM5 (full length)

### 1-1. CEACAM5 (full length) antigen sequencing and protein purification

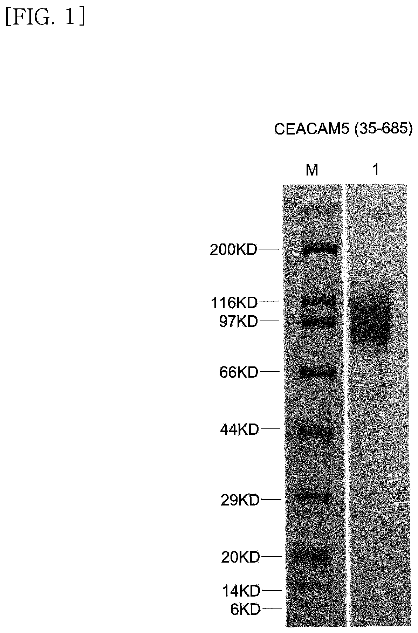

Hereinafter, CEACAM5 antigen sequencing and protein purification for the production of anti-CEACAM5 antibodies according to an exemplary embodiment of the present disclosure will be described in detail with reference to FIG. 1.

FIG. 1 is a diagram showing results obtained by purifying a CEACAM5 recombinant protein (full length) produced in cells after transfection into CHO-K1 cells according to an exemplary embodiment of the present disclosure.

Specifically, the amino acid sequence and DNA sequence (SEQ ID NO: 47, CEACAM5 full length amino acid sequence, GeneBank NM_001291484.3) of an extracellular protein domain (ECD) of a human CEACAM5 protein were confirmed, and the corresponding DNA gene was synthesized (Genscript Probio).

The synthesized corresponding nucleotide sequence containing an amino acid His tag at a carbonyl-terminal of the protein amino acid sequence was transiently transfected into an Expi293 cell line, which was a cell line derived from human embryonic kidney, according to the manufacturer's instructions (Expi293 Expression System, ThermoFisher Scientific).

Thereafter, the CEACAM5 full-length recombinant protein was purified from the expressed culture supernatant using Ni Sepharose affinity chromatography according to the manufacturer's instructions (GE Healthcare).

### 1-2. Identification of production of in vivo CEACAM5 (full length) antibodies

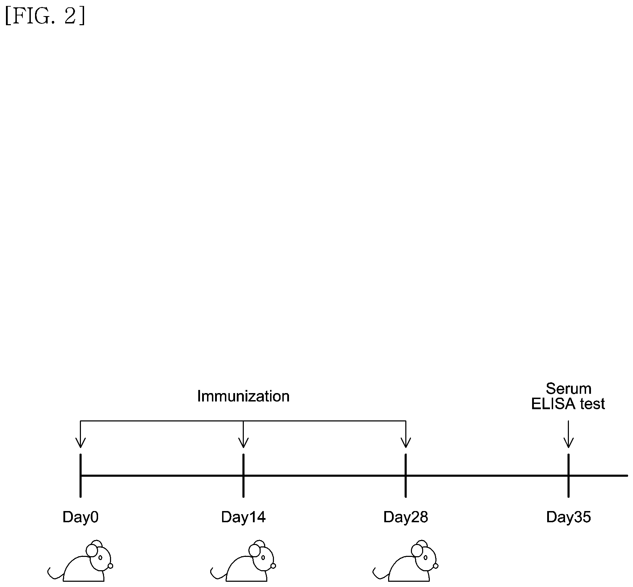

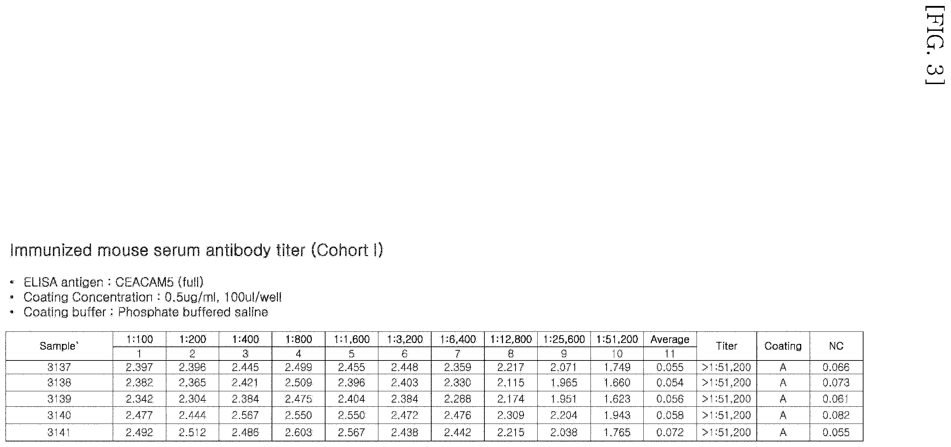

Hereinafter, a process of producing in vivo CEACAM5 antibodies in a mouse model will be described in detail through FIGS. 2 and 3.

FIG. 2 is a diagram illustrating a process of immunizing mice with a CEACAM5 recombinant protein (full length) according to an exemplary embodiment of the present disclosure.

FIG. 3 is a diagram illustrating a result of immunizing mice with a CEACAM5 recombinant protein (full length) according to an exemplary embodiment of the present disclosure.

First, referring to FIG. 2, a high binding 96-well microplate produced as described above was coated with 100 µl/well of the CEACAM5 full-length recombinant protein at a concentration of 0.5 µl/ml, and then incubated at 4°C for 16 hours. Thereafter, the coated microplate was washed four times with PBS-T (1x PBS, 0.5% Tween-20), and then added with PBS-T containing 3% BSA and incubated at room temperature for 1 hour. After washing four times with PBS-T, the serum collected from the mice was diluted by each dilution factor, added to the microplate at 100 µl/well, and reacted at room temperature for 2 hours. Thereafter, after washing four times using PBS-T, 100 µl/well of HRP-anti-mouse IgG, FcR (Jackson ImmunoResearch) binding to a mouse antibody was added and reacted at room temperature for 1 hour, and then washed four times with PBS-T and added with 100 µl/ml of a TMB substrate solution and reacted at room temperature for 10 minutes. Finally, 100 µl/ml of a stop solution was added, and the absorbance was measured at 450 nm.

As a result, referring to FIG. 3, it was confirmed that the CEACAM5 antibodies were well produced in all 5 mice.

### 1-3. Selection of parental clones binding to CEACAM5 full-length protein

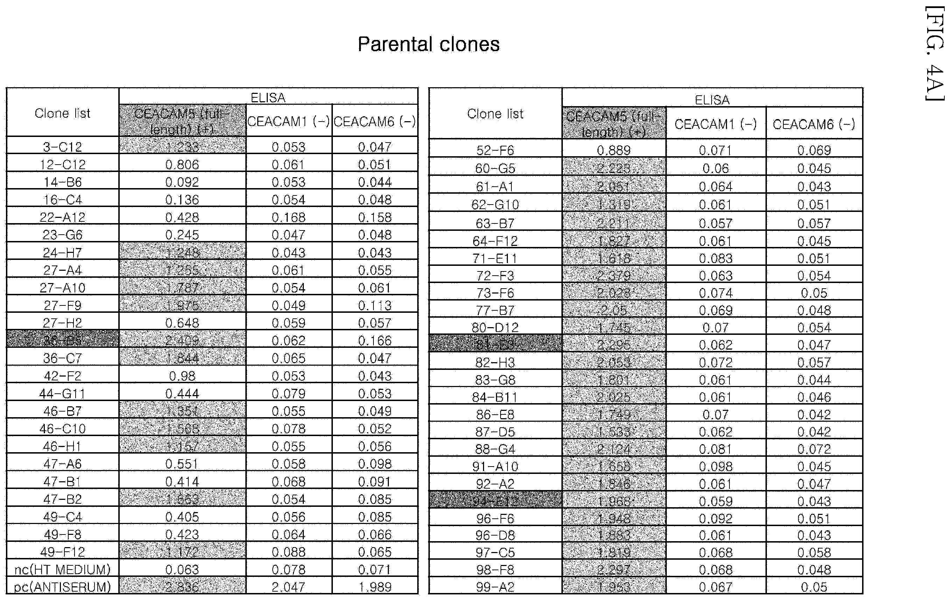

Hereinafter, selection of parental clones for selecting antibodies specifically binding to CEACAM5 will be described in detail with reference to FIG. 4A.

FIG. 4A is a diagram illustrating results of selecting parental clones that bind to a CEACAM5 full length protein.

By fusing mouse myeloma cells, SP2/0 cells, and B cells obtained from mice, hybridoma cells were prepared and incubated separately in 96-well plates (10 plates). Using the culture solution obtained from the parental clones (1 parental clone/well) incubated separately, clones binding to full-length CEACAM5 were identified by ELISA, and it was also confirmed whether the clones non-specifically bound to CEACAM1 and CEACAM6, which had similar homology. After selecting parental clones binding to the CEACAM5 full-length protein without binding to other CEACAM proteins, subclones were selected.

Specifically, a high binding 96-well microplate was coated with 100 µl/well of CEACAM5, CEACAM1, and CEACAM6 full length recombinant proteins at a concentration of 0.5 µl/ml, and then incubated at 4°C for 16 hours. Thereafter, the coated microplate was washed four times with PBS-T (1x PBS, 0.5% Tween-20), and then added with PBS-T containing 3% BSA and incubated at room temperature for 1 hour (blocking). After washing four times using PBS-T, the serum collected from the mice was diluted by each dilution factor, added to the microplate at 100 µl/well, and reacted at room temperature for 2 hours. After washing four times using PBS-T, HRP-anti-mouse IgG, FcR (Jackson ImmunoResearch) binding to mouse antibodies was added at 100 µl/well and reacted at room temperature for 1 hour. After washing four times with PBS-T, a TMB substrate solution was added at 100 µl/ml and reacted at room temperature for 10 minutes. Finally, 100 µl/ml of a stop solution was added, and the absorbance was measured at 450 nm.

As a result, referring to FIG. 4A, clones showing high binding affinity to CEACAM5 (full-length) and low binding affinity to CEACAM 1 and CEACAM 6 were selected from the parental clones, and as a result, it was confirmed that parental clones including 36-B5, 81-E3, and 94-E12 showed high binding affinity specifically to CEACAM5 (full-length).

### 1-4. Selection of subclones binding to CEACAM5 full length protein

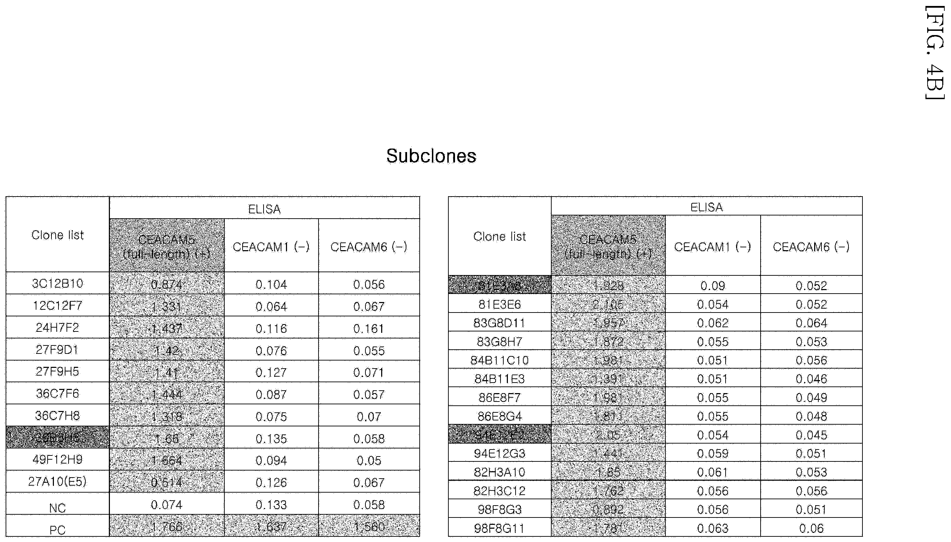

Hereinafter, selection of subclones for selecting antibodies specifically binding to CEACAM5 will be described in detail with reference to FIG. 4B.

FIG. 4B is a diagram illustrating results of selecting subclones that bind to a CEACAM5 full length protein.

As in Example 1-3, some of the selected parental clones were selected and isolated into monoclones and incubated in a 96-well plate. Using the culture solution obtained from the monoclones, clones binding to CEACAM5 full length were identified by ELISA, and it was also confirmed whether the clones non-specifically bound to CEACAM1 and CEACAM6, which had similar homology.

Clones binding to the CEACAM5 full length protein without binding to the CEACAM1 and CEACAM6 proteins were selected by conducting an experiment in the same manner as in Example 1-3.

As a result, referring to FIG. 4B, it was confirmed that all subclones including 36B5H8, 81E3A8, and 94E12E2 derived from the selected parental clones bound to the CEACAM5 full length protein without binding to the CEACAM 1 and CEACAM 6 proteins.

### 1-5. Confirmation of binding capacity of antibodies to CHO-K1, CHO-K1-CEACAM5 (full), and CHO-K1-CEACAM5 (A3-B3) cells

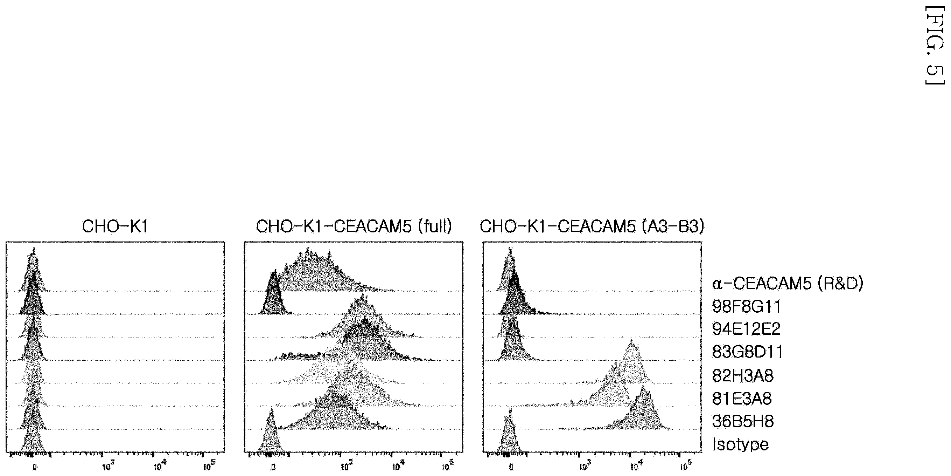

Hereinafter, results of confirming binding capacity of antibodies purified from CHO-K1, CHO-K1-CEACAM5 (full), and CHO-K1-CEACAMS (A3-B3) cells and six single-antibody clones (hybridoma cells) will be described with reference to FIG. 5.

FIG. 5 is a diagram illustrating binding capacity of antibodies purified from CHO-K1, CHO-K1-CEACAM5 (full), and CHO-K1-CEACAM5 (A3-B3) cells and six single-antibody clones (hybridoma cells).

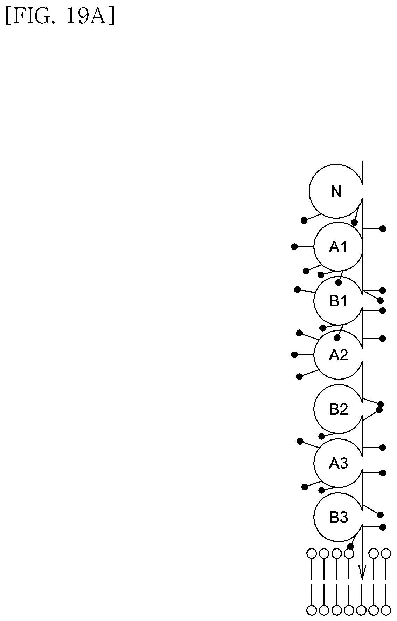

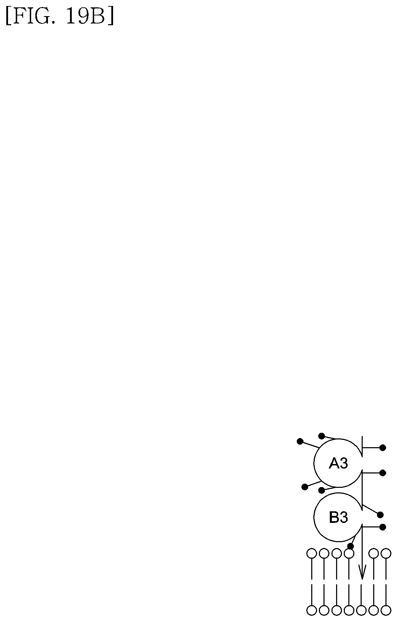

To select subclones binding to the CEACAM5 A3 and B3 domains (FIG. 19B), which were domains predicted to remain in the membrane protein after CEACAM5 was cleaved, antibodies purified from six single-antibody clones (hybridoma cells) were added at 1 µg/100 µl each to CHO-K1, CHO-K1-CEACAM5 (full), and CHO-K1-CEACAM5 (A3-B3) cells and reacted at 4°C for 30 minutes. At this time, mouse IgG was used as a negative control and a R&D product [FAB41281P] was used as a positive control. After washing with 2 ml of 1X PBS, 1 µl/100 µl of PE-anti-mouse IgG was added to each sample and reacted at 4°C for 30 minutes in a light-blocking state. After washing with 2 ml of 1X PBS, 300 µl of PBS was added to each sample, and the binding of antibodies was confirmed using a flow cytometer.

As a result, as shown in FIG. 5, it was confirmed that among subclones 98F8G11, 94E12E2, 83G8D11, 82H3A8, 81E3A8, and 36B5H8, 94E12E2, 83G8D11, 82H3A8, 81E3A8, and 36B5H8 had high binding affinity to the CEACAM5 full-length protein, and among them, 82H3A8, 81E3A8, and 36B5H8 had high binding affinity to the A3 and B3 domains of CEACAMS.

### Example 2: Selection of antibodies binding to CEACAM5 (A3 and B3 domains)

### 2-1. In vivo CEACAM5 (A3 and B3 domains) antigen sequencing and protein purification

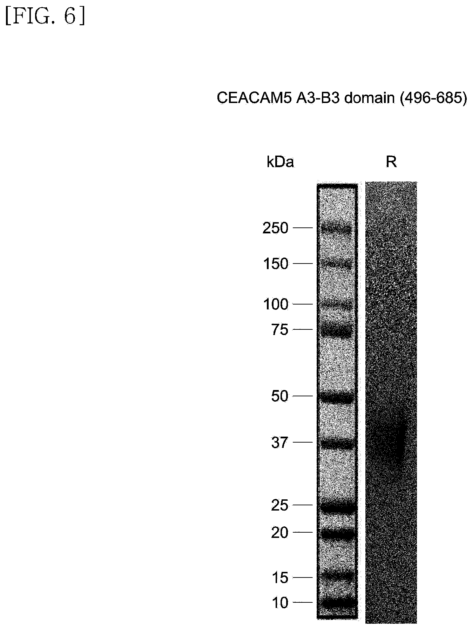

Hereinafter, CEACAM5 antigen sequencing and protein purification for producing anti-CEACAM5 antibodies according to an exemplary embodiment of the present disclosure will be described in detail with reference to FIG. 6.

FIG. 6 is a diagram illustrating results obtained by purifying a CEACAM5 recombinant protein (A3 and B3 domains) produced in cells after transfection into CHO-K1 cells according to an exemplary embodiment of the present disclosure.

In order to obtain the CEACAM5 recombinant protein (A3 and B3 domains), in order to develop antibodies that recognized the membrane protein remaining on the cell membrane surface after cleavage of CEACAM5, a gene containing only the A3 and B3 domains (SEQ ID NO: 48, CEACAM5 A3-B3 amino acid sequence) in the amino acid sequence and DNA sequence of the extracellular protein domain (ECD) of human CEACAM5 protein was synthesized (Genscript Probio).

The synthesized corresponding nucleotide sequence containing an amino acid His tag at a carbonyl-terminal of the protein amino acid sequence was transiently transfected into an Expi293 cell line, which was a cell line derived from human embryonic kidney, according to the manufacturer's instructions (Expi293 Expression System, ThermoFisher Scientific).

The CEACAM5 (A3-B3) protein was purified from the expressed culture supernatant using Ni Sepharose affinity chromatography according to the manufacturer's instructions (GE Healthcare).

### 2-2. Confirmation of production of in vivo CEACAM5 (A3 and B3 domains) antibodies

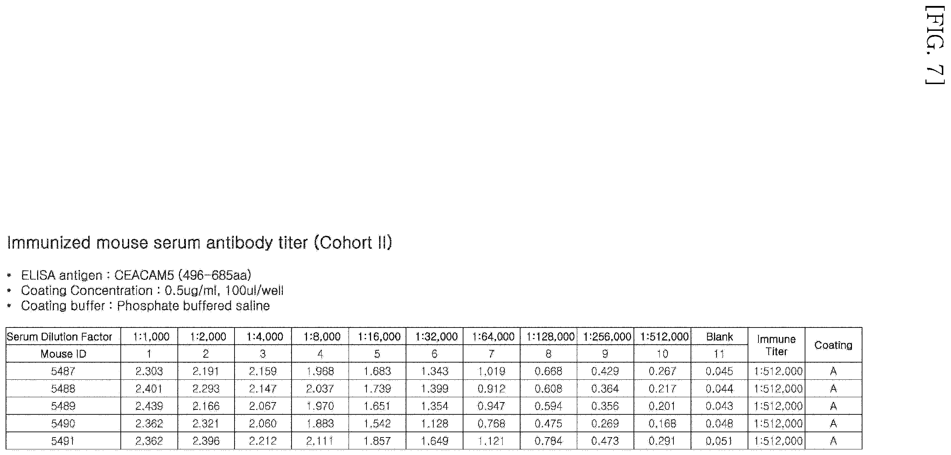

Hereinafter, a process of producing in vivo CEACAM5 (A3 and B3 domains) antibodies in a mouse model will be described in detail with reference to FIGS. 2 and 7.

FIG. 2 is a diagram illustrating a process of immunizing mice with a CEACAM5 recombinant protein (full length) according to an exemplary embodiment of the present disclosure.

FIG. 7 is a diagram illustrating results of immunizing mice with a CEACAM5 recombinant protein (A3 and B3 domains) according to an exemplary embodiment of the present disclosure.

Referring back to FIG. 2, the CEACAM5 recombinant protein (A3 and B3 domains) was immunized in mice in the same manner as the process of immunizing the mice with the CEACAM5 recombinant protein (full length) illustrated in FIG. 2.

Specifically, the CEACAM5 (A3-B3) protein produced as described above was immunized in the mice three times at 14-day intervals, and one week after the final immunization, serum was collected to confirm whether antibodies to CEACAM5 (A3-B3) were produced in the mouse body. The collected mouse serum was diluted 1,000 to 512,000-fold with PBS, and the degree of CEACAM5 antibody production was confirmed through ELISA (ELISA antigen: CEACAM5 (496-685aa)/ Coating Concentration: 0.5 µg/ml, 100 µl/well/ Coating buffer: Phosphate buffered saline).

Referring to FIG. 7, it was confirmed that CEACAM5 antibodies were well produced in all five mice immunized.

### 2-3. Selection of parental clones binding to CEACAM5 A3 and B3 domain proteins

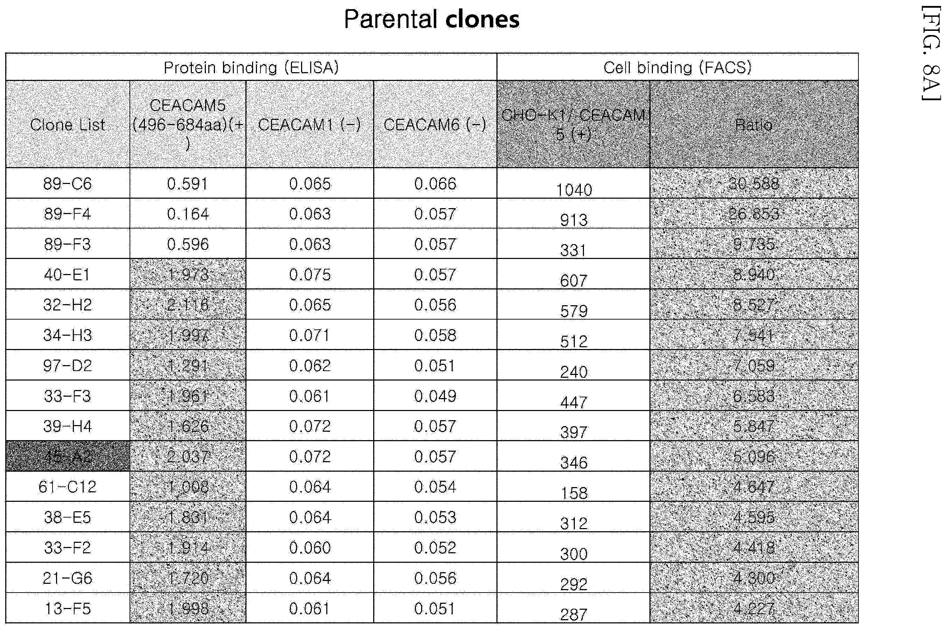

Hereinafter, selection of parental clones binding to CEACAM5 A3 and B3 domain proteins will be described in detail with reference to FIG. 8A.

FIG. 8A is a diagram illustrating results of selecting parental clones binding to CEACAM5 A3 and B3 domain proteins.

To select parental clones binding to CEACAM5 A3 and B3 domain proteins, hybridoma cells were first prepared by fusing SP2/0 cells, which were mouse myeloma cells, with B cells obtained from mice, and then incubated separately in 96-well plates (10 plates). Using the culture solution obtained from the parental clones (1 parental clone/well) incubated separately, clones binding to the CEACAM5 A3 and B3 domains were identified by ELISA, and it was confirmed whether the clones non-specifically bound to CEACAM1 and CEACAM6 having similar homology, and parental clones binding to a CEACAM5 A3-B3 protein without binding to CEACAM 1 and CEACAM 6 proteins were selected.

Specifically, a high binding 96-well microplate was coated with 100 µl/well of CEACAM5, CEACAM1, and CEACAM6 full length recombinant proteins at a concentration of 0.5 µl/ml, and then incubated at 4°C for 16 hours. Thereafter, the coated microplate was washed four times with PBS-T (1x PBS, 0.5% Tween-20), and then added with PBS-T containing 3% BSA and incubated at room temperature for 1 hour (blocking). After washing four times with PBS-T, the serum collected from the mice was diluted by each dilution factor, added to the microplate at 100 µl/well, and reacted at room temperature for 2 hours. After washing four times with PBS-T, HRP-anti-mouse IgG, FcR (Jackson ImmunoResearch), which bound to mouse antibodies, were added at 100 µl/well and reacted at room temperature for 1 hour. Thereafter, after washing four times with PBS-T, a TMB substrate solution was added at 100 µl/ml, reacted at room temperature for 10 minutes, and then added with 100 µl/ml of a stop solution and the absorbance was measured at 450 nm.

As a result, referring to FIG. 8A, it was confirmed that the parental clones including 45-A2 showed high binding affinity specifically to the CEACAM5 A3-B3 protein and low binding affinity to CEACAM 1 and CEACAM 6 proteins.

### 2-4. Selection of subclones binding to CEACAM5 protein

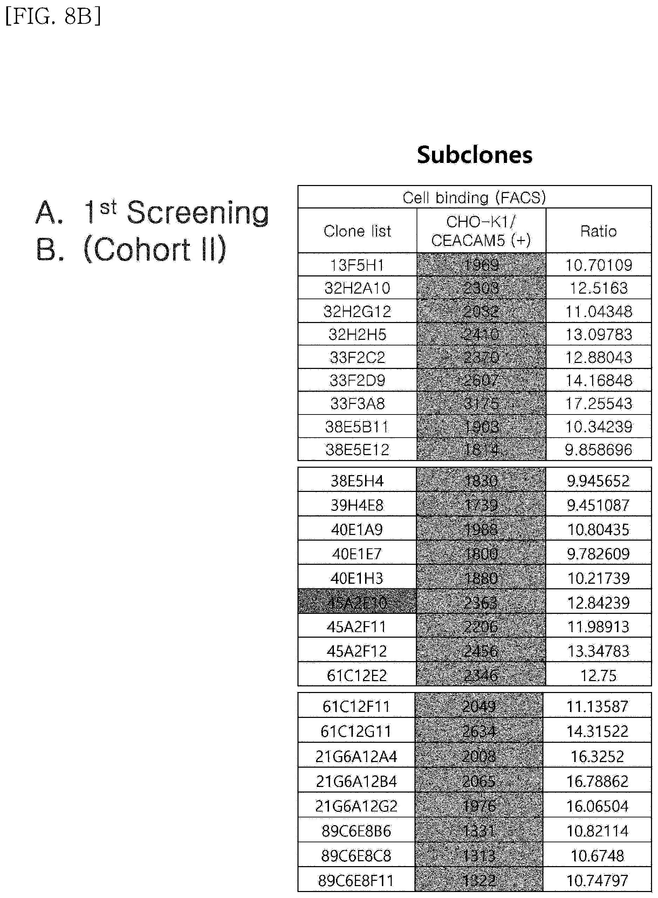

Hereinafter, selection of subclones binding to CEACAM5 A3 and B3 domain proteins will be described in detail with reference to FIG. 8B.

FIG. 8B is a diagram illustrating results of selecting subclones binding to CEACAM5 A3 and B3 domain proteins.

As in Example 2-3, some of the selected parental clones were selected and isolated into monoclones and incubated in a 96-well plate. Using the culture solution obtained from the monoclones, clones binding to CEACAM5 A3 and B3 domain proteins were identified by ELISA, and it was also confirmed whether the clones non-specifically bound to CEACAM1 and CEACAM6, which had similar homology.

Clones binding to the CEACAM5 A3 and B3 domain proteins without binding to the CEACAM1 and CEACAM6 proteins were selected by conducting an experiment in the same manner as in Example 2-3.

As a result, referring to FIG. 8B, it was confirmed that all subclones including 45A2E10 derived from the selected parental clones specifically bound to the CEACAM5 A3 and B3 domain proteins without binding to the CEACAM 1 and CEACAM 6 proteins.

### 2-5. Confirmation of binding capacity of CEACAM5 antibodies purified by monoclones to CEACAM5 full-length protein

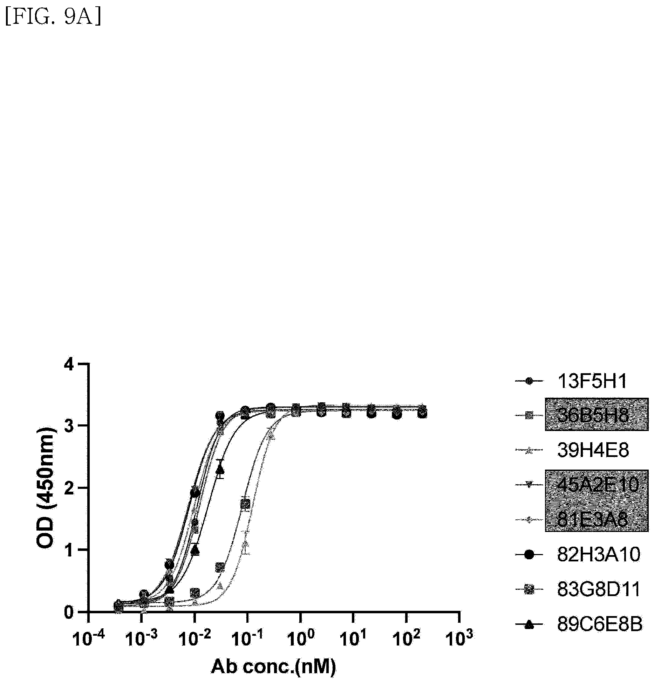

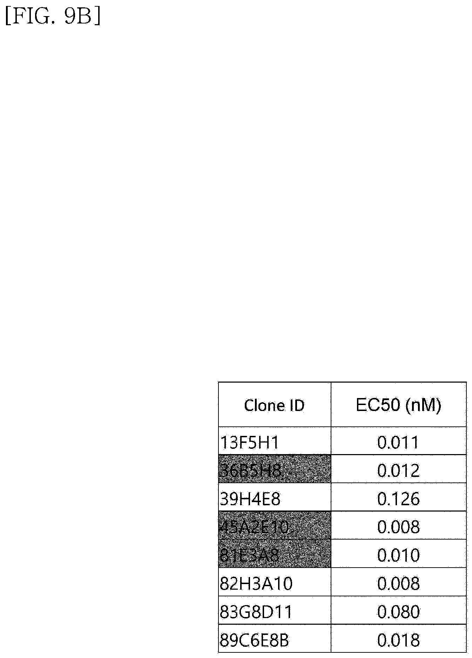

Hereinafter, the binding capacity of CEACAM5 antibodies purified by monoclones according to an exemplary embodiment of the present disclosure to a CEACAM5 full-length protein will be described in detail with reference to FIGS. 9A and 9B.

FIGS. 9A and 9B are diagrams illustrating the binding capacity of CEACAM5 antibodies purified by monoclones according to an exemplary embodiment of the present disclosure to a CEACAM5 full-length protein.

To confirm the binding capacity of the CEACAM5 antibodies purified by monoclones to the CEACAM5 full-length protein, first, 30 µl of a CEACAM5 protein (Acrobiosystems) was dispensed per well of a 384-well microplate (Corning) at a concentration of 0.5 µg/ml, and incubated at 4°C for 16 hours, and then washed four times with 100 µL/well of PBST (DPBS + 0.05% Tween 20, Teknova). Then, the microplate was added with 80 µL/well of 1X PBS buffer containing 3% FBS, incubated at room temperature for 2.5 hours, and then washed four times with 100 µL/well of PBST in the same manner as above. At this time, the antibodies were prepared by diluting 3-fold to a concentration of 200 nM to 0.0004 nM with DPBS (Bining buffer) containing 0.5% Fetal Bovin Serum (FBS, Cytiva). The antibodies prepared as such were added to a CEACAM5-coated plate at 30 µL/well and incubated at room temperature for 1 hour. Thereafter, the plate was washed, and HRP (horseradish peroxidase) anti-mouse IgG Fcγ (secondary antibody, Jackson ImmunoResearch) was diluted to a concentration of 0.16 µg/mL (1 : 5000) in a binding buffer, added to the plate at 30 µL/well, and incubated at room temperature for 1 hour. Thereafter, the plate was washed in the same manner, added with a TMB (3,3',5,5'-tetramethylbenzidine, Invitrogen) substrate at 30 µL/well, and then incubated in the dark at room temperature for 10 minutes. Finally, the plate was added with 30 µL/well of a 0.16 M sulfuric acid (stop solution, Invitrogen) solution, and incubated in the dark for 10 minutes to stop the reaction, and the absorbance was measured at 450 nm (Synergy htx multimode reader, Biotek).

As a result, as illustrated in FIGS. 9A and 9B, it was confirmed that among the CEACAM5 antibodies purified by the monoclones of the present disclosure, the binding capacity of the 36B5H8, 45A2E10, and 81E3A8 antibodies was particularly high.

### 2-6. Confirmation of binding capacity of selected CEACAM5 antibodies to A549 cell line expressing CEACAM5

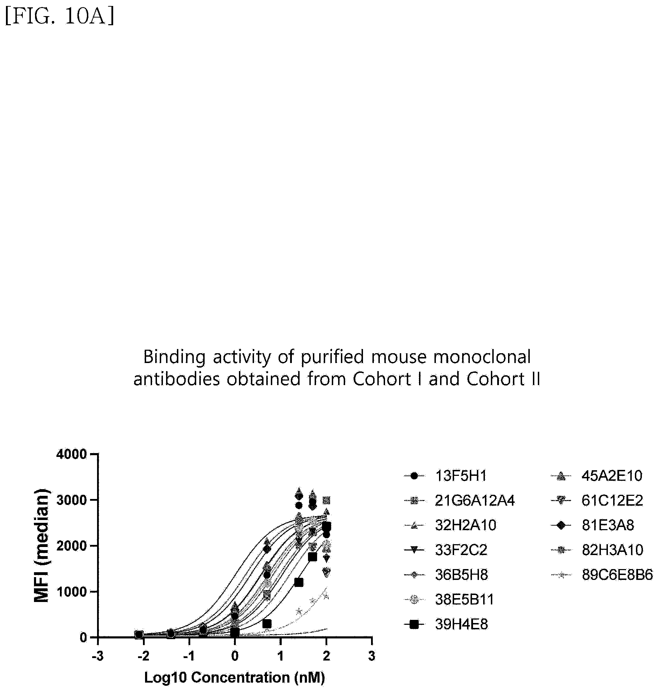

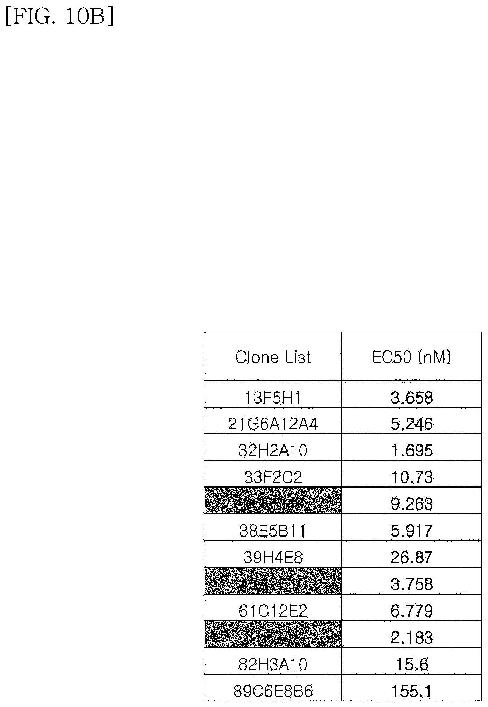

Hereinafter, the binding capacity of CEACAM5 antibodies purified by monoclones according to an exemplary embodiment of the present disclosure to an A549 cell line expressing CEACAM5 will be described in detail with reference to FIGS. 10A and 10B.

FIGS. 10A and 10B are diagrams illustrating the binding capacity of CEACAM5 antibodies purified by monoclones according to an exemplary embodiment of the present disclosure to an A549 cell line expressing CEACAM5.

First, the A549 cell line (A549-CEACAM5) expressing CEACAM5 was mixed with mouse antibodies at various concentrations (0.008 nM to 100 nM) and 1X PBS containing 3% FBS, and then incubated at 4°C for 2 hours. After washing twice with 1X PBS, a secondary antibody of goat anti-mouse IgG (Jackson ImmunoResearch) conjugated with Alexa Flour 488 was mixed at a concentration of 5 µg/ml in 1X PBS containing 3% FBS, and incubated at 4°C for 1 hour. After washing twice with Cold PBS, fluorescence was measured by a flow cytometer (BD FACS Verse II) and MFI was analyzed using Flowjo software.

As a result, referring to FIGS. 10A and 10B, it was confirmed that the binding capacity of 81E3A8 and 45A2E10 antibodies to the A549 cell line was particularly excellent.

### 2-7. Confirmation of blocking effect of selected CEACAM5 antibodies due to soluble CEACAM5

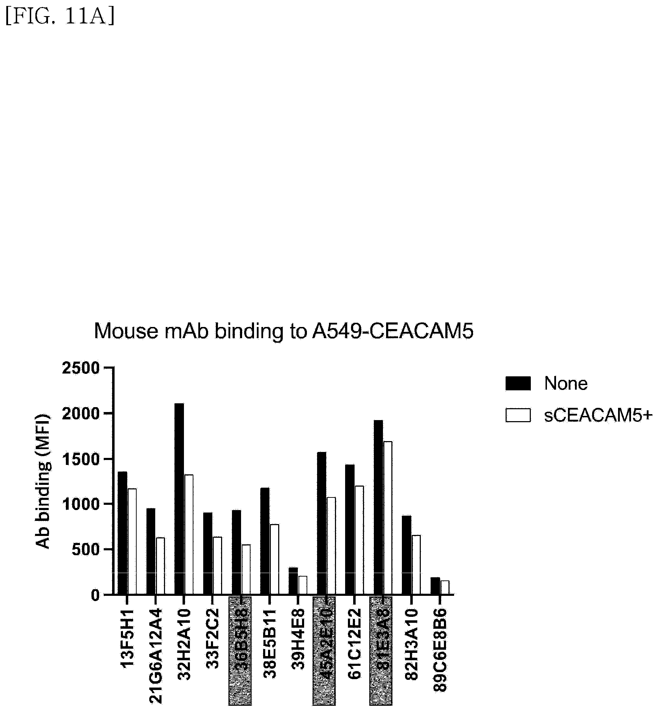

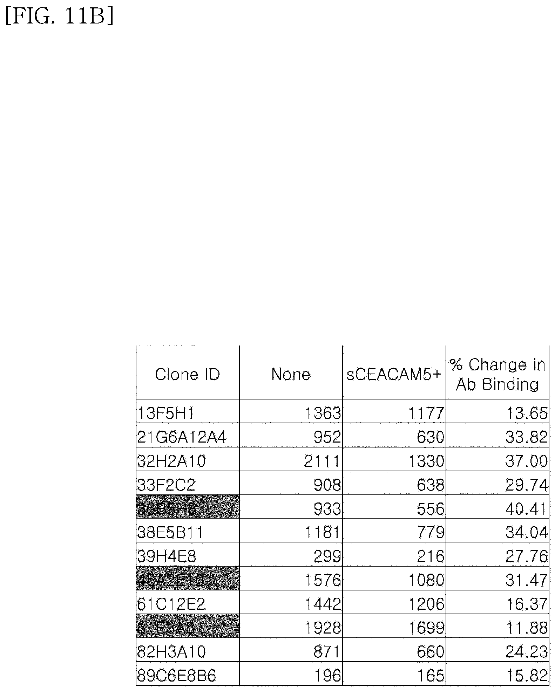

Hereinafter, the blocking effects of the antibodies according to an exemplary embodiment of the present disclosure due to soluble CEACAM5 will be described in detail through FIGS. 11A and 11B.

FIGS. 11A and 11B are diagrams illustrating blocking effects of CEACAM5 antibodies due to soluble CEACAM5 according to an exemplary embodiment of the present disclosure.

In order to confirm the blocking effects of the CEACAM5 antibodies due to soluble CEACAM5, first, an A549 cell line (A549-CEACAM5) expressing CEACAM5 was mixed with mouse antibodies at various concentrations (0.008 nM to 100 nM) in 1X PBS containing 3% FBS, and then added with cancer patient's serum containing soluble CEACAM5 at a concentration of 100 ng/ml, incubated at 4°C for 2 hours, and washed twice with 1X PBS. Thereafter, the secondary antibody of goat anti-mouse IgG (Jackson ImmunoResearch) conjugated with Alexa Flour 488 was mixed at a concentration of 5 µg/ml in 1X PBS containing 3% FBS, incubated at 4°C for 1 hour, washed twice with Cold PBS, and then fluorescence was measured by a flow cytometer (BD FACS Verse II) and MFI was analyzed using Flowjo software. These values show how much the tested antibodies bound to the surface of the A549 cell line expressing CEACAM5 on the cell surface. The MFI values of a sample (Sceacam5+) containing cancer patient's serum containing soluble CEACAM5 and a sample (None) without the cancer patient's serum were compared to express the blocking effect due to soluble CEACAM5 as a % ratio.

Referring to FIGS. 11A and 11B, it was found that the CEACAM5 antibodies according to an exemplary embodiment of the present disclosure had a blocking effect due to soluble CEACAM5 of less than 50% in a sample containing soluble CEACAM5, and in particular, in the case of the 81E3A8 antibody, the % ratio for the blocking effect due to soluble CEACAM5 was 11.88, and thus it was confirmed that high binding capacity to the A549 cell line expressing CEACAM5 was exhibited even in the sample containing soluble CEACAM5.

That is, it was confirmed that the CEACAM5 antibodies according to an exemplary embodiment of the present disclosure did not virtually inhibit the binding capacity to CEACAM5 located on tumor cells even in the sample containing soluble CEACAM5, and thus it was confirmed that the antibodies specifically bound to CEACAM5 present on the surface of tumor cells without substantially binding to soluble CEACAM5. This shows that the CEACAM5 antibodies according to the present disclosure may be used in a more effective and efficient manner for tumor treatment, which provides an important advantage in a cancer treatment method using CEACAM5 antibodies.

### 3-1. Humanization of antibody 81E3A8

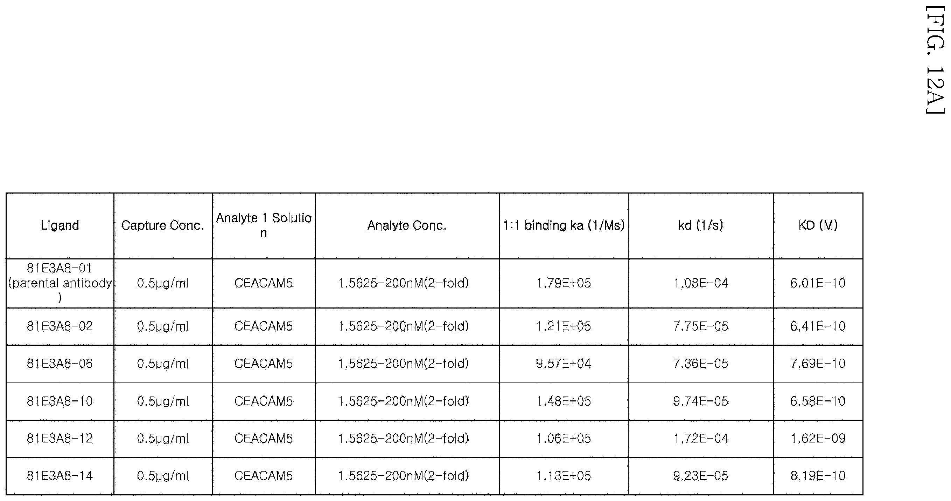

Hereinafter, an antibody 81E3A8 produced by performing a humanization process according to an exemplary embodiment of the present disclosure will be described in detail through FIG. 12A.

FIG. 12A is a diagram illustrating analysis of the binding affinity of an antibody 81E3A8 produced by performing a humanization process according to an exemplary embodiment of the present disclosure.

In order to analyze the binding affinity of the antibody 81E3A8 produced by performing the humanization process, the humanization process of the 81E3A8 clones was performed to produce humanized antibodies, and among them, five antibodies 81E3A8-02, 81E3A8-06, 81E3A8-10, 81E3A8-12, and 81E3A8-14 with excellent binding affinity were selected as illustrated in FIG. 12A. At this time, 81E3A8-01 corresponded to an antibody produced with a mouse sequence as a control, and the humanized antibodies of the clonal antibodies were produced by combining the same CDR 1 to 3 sequences and different framework sequences of the clonal antibodies, as shown in Tables 1 to 6 below.

**[Table 1]**

| CDR sequences of humanized 81E3A8 antibody | | | | |
|---|---|---|---|---|
| Antibody | Chain type | | sequence | Seq no. |
| humanized 81E3A8 antibody | VH | CDR 1 | NFGMN | 1 |
| | | CDR 2 | WINTYTGKPTYDDDFKG | 2 |
| | | CDR 3 | EAGKDYAMDY | 3 |
| | VL | CDR 1 | SASSSVTFMH | 4 |
| | | CDR 2 | DTSKLAS | 5 |
| | | CDR 3 | QQWNNYPWT | 6 |

**[Table 2]**

| Framework sequences of humanized 81E3A8-02 clonal antibody | | | |
|---|---|---|---|
| Antibody | Chain type | sequence | Seq no. |
| humanized 81E3A8-02 antibody | VH | QIQLVQSGSELKKPGASVKVSCKASGY TFTNFGMNWVRQAPGQGLEWMGWINTY TGKPTYDDDFKGRFVFSLDTSVSTAYL | 7 |
| | | QISSLKAEDTAVYYCAREAGKDYAMDY WGQGTLVTVSS | |
| | VL | EIVLTQSPATLSLSPGERATLSCSASS SVTFMHWYQQKPGQAPRRLIYDTSKLA SGIPARFSGSGSGTDYTLTISSLEPED AAVYYCQQWNNYPWTFGGGTKVEIK | 8 |

**[Table 3]**

| Framework sequences of humanized 81E3A8-06 clonal antibody | | | |
|---|---|---|---|
| Antibody | Chain type | sequence | Seq no. |
| humanized 81E3A8-06 antibody | VH | QIQLVQSGSELKKPGASVKVSCKASG YTFTNFGMNWVRQAPGQGLEWMGWIN TYTGKPTYDDDFKGRFVFSLDTSVST AYLQISSLKAEDTAVYYCAREAGKDY AMDYWGQGTLVTVSS | 7 |
| | VL | DIQLTQSPSTLSASVGDRVTITCSAS SSVTFMHWYQQKPGKAPKRLIYDTSK LASGVPSRFSGSGSGTEYTLTISSLQ PDDAATYYCQQWNNYPWTFGGGTKVE IK | 9 |

**[Table 4]**

| Framework sequences of humanized 81E3A8-10 clonal antibody | | | |
|---|---|---|---|
| Antibody | Chain type | sequence | Seq no. |
| humanized 81E3A8-10 antibody | VH | QIQLVQSGSELKKPGASVKVSCKASGYTF TNFGMNWVRQAPGQGLEWMGWINTYTGKP TYDDDFKGRFVFSLDTSVSTAYLQISSLK AEDTAVYYCAREAGKDYAMDYWGQGTLVT VSS | 7 |
| | VL | EIVLTQSPATLSVSPGERATLSCSASSSV TFMHWYQQKPGQAPRRLIYDTSKLASGIP ARFSGSGSGTEYTLTISSMQSEDAAVYYC QQWNNYPWTFGGGTKVEIK | 10 |

**[Table 5]**

| Framework sequences of humanized 81E3A8-12 clonal antibody | | | |
|---|---|---|---|
| Antibody | Chain type | sequence | Seq no. |
| humanized 81E3A8-12 antibody | VH | QIQLVQSGAEVKKPGASVKVSCKASGYT FTNFGMNWVRQAPGQGLEWMGWINTYTG KPTYDDDFKGRVTMTLDTSTSTAYMELR SLRSDDMAVYYCAREAGKDYAMDYWGQG TLVTVSS | 11 |
| | VL | EIVLTQSPATLSVSPGERATLSCSASSS VTFMHWYQQKPGQAPRRLIYDTSKLASG IPARFSGSGSGTEYTLTISSMQSEDAAV YYCQQWNNYPWTFGGGTKVEIK | 10 |

**[Table 6]**

| Framework sequences of humanized 81E3A8-14 clonal antibody | | | |
|---|---|---|---|
| Antibody | Chain type | sequence | Seq no. |
| humanized 81E3A8-14 antibody | VH | QIQLVQSGSELKKPGASVKVSCKASGYTF TNFGMNWVRQAPGQGLEWMGWINTYTGKP TYDDDFKGRFVFSLDTSVSTAYLQISSLK AEDTAVYYCAREAGKDYAMDYWGQGTLVT VSS | 7 |
| | VL | DIQLTQSPSSLSASVGDRVTITCSASSSV TFMHWYQQKPGKAPKRLIYDTSKLASGVP SRFSGSGSGTDYTLTISSLQPEDAATYYC QQWNNYPWTFGGGTKVEIK | 12 |

The binding affinity analysis of the humanized antibodies was performed using Biacore 8K equipment. First, the antibodies were bound to a protein A chip at a concentration of 0.5 µg/ml, and then reacted with a CEACAM5 recombinant protein at various concentrations of 1.5625 to 200 nM to analyze the binding affinity of the antibodies above, which was illustrated in FIG. 12A.

### 3-1. Confirmation of binding capacity of antibody 81E3A8 produced by performing humanization process

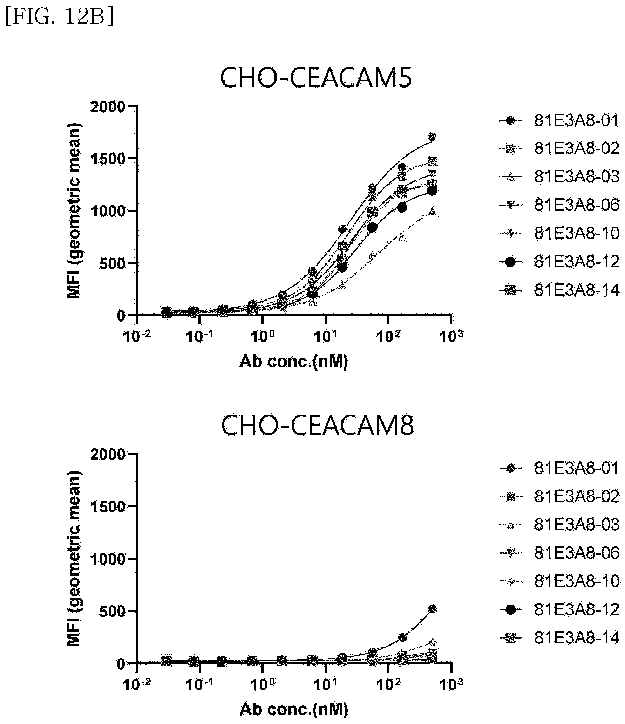

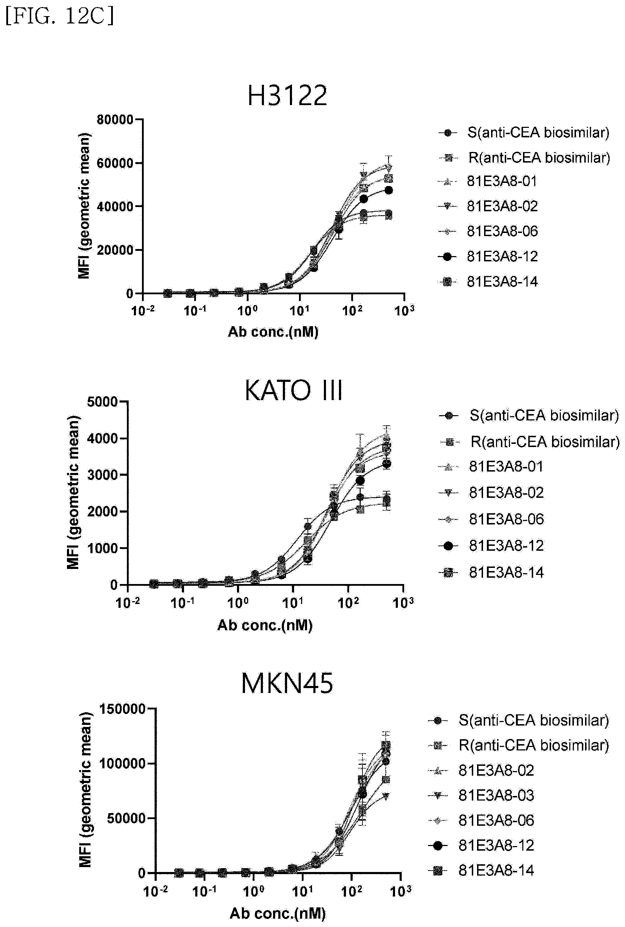

Hereinafter, the binding capacity of an antibody 81E3A8 produced by performing a humanization process according to an exemplary embodiment of the present disclosure to a CHO-k1 (CHO-CEACAM5) cell line expressing CEACAM5 and a CHO-k1 (CHO-CEACAM8) cell line expressing CEACAM8 will be described in detail with reference to FIGS. 12B and 12C.

FIG. 12B is a diagram illustrating comparing the binding capacity of an antibody 81E3A8 produced by performing a humanization process according to an exemplary embodiment of the present disclosure to a CHO-k1 (CHO-CEACAM5) cell line expressing CEACAM5 and a CHO-k1 (CHO-CEACAM8) cell line expressing CEACAM8.

FIG. 12C is a diagram illustrating the binding capacity of an antibody 81E3A8 produced by performing a humanization process according to an exemplary embodiment of the present disclosure to H3122, KATO III, and MKN45 cell lines expressing CEACAM5.

In order to confirm the binding capacity of the antibody 81E3A8 produced by performing the humanization process according to an exemplary embodiment of the present disclosure to the CHO-k1 (CHO-CEACAM5) cell line expressing CEACAM5 and the CHO-k1 (CHO-CEACAM8) cell line expressing CEACAM8, first, the CHO-k1 (CHO-CEACAM5) cell line expressing CEACAM5 and the CHO-k1 (CHO-CEACAM8) cell line expressing CEACAM8 were dispensed into a 96-well plate at 3 × 10⁶ cells/well. Thereafter, humanized mAbs at various concentrations (0.025 nM to 500 nM) were diluted with a DPBS solution, dispensed into a CHO-CEACAM5 cell plate at each 50 µL/well, and then incubated at 4°C for 20 minutes. The plate was washed twice with 250 µL/well of Cold DPBS. (centrifugation at 1500 RPM and 4°C for 3 minutes) Then, the cells were dispensed with 50 µL/well of a solution in which a goat anti-human IgG Fcγ secondary antibody (Jackson ImmunoResearch) conjugated with Alexa Flour 647 was diluted with DPBS to a concentration of 15 µg/mL, and then the plate was wrapped with foil to block light and incubated at 4°C for 20 minutes, and washed twice with 250 µL/well of Cold DPBS. (centrifugation at 1500 RPM and 4°C for 3 minutes) Finally, 250 µL/well of Cold DPBS was dispensed, and the fluorescence was measured using a flow cytometer (MACSQuant^{®} Analyzer 10, Miltenyi Biotec) and the MFI (geometric mean) was analyzed using BD Flowjo.

As a result, referring to FIG. 12B, it was confirmed that all five antibodies 81E3A8-02, 81E3A8-06, 81E3A8-10, 81E3A8-12, and 81E3A8-14 produced by performing the humanization process according to an exemplary embodiment of the present disclosure had low binding capacity to the cell line expressing CEACAM8, but high binding capacity to the CHO-k1 (CHO-CEACAM5) cell line expressing CEACAM5.

In addition, as a result of confirming the binding capacity to H3122, KATO III, and MKN45 cell lines expressing CEACAM5 using the above method, it was confirmed that all of the antibodies 81E3A8-02, 81E3A8-06, 81E3A8-12, and 81E3A8-14 produced by performing the humanization process according to an exemplary embodiment of the present disclosure showed high binding capacity.

### 3-2. Confirmation of binding capacity of antibody 45A2E10 produced by performing humanization process

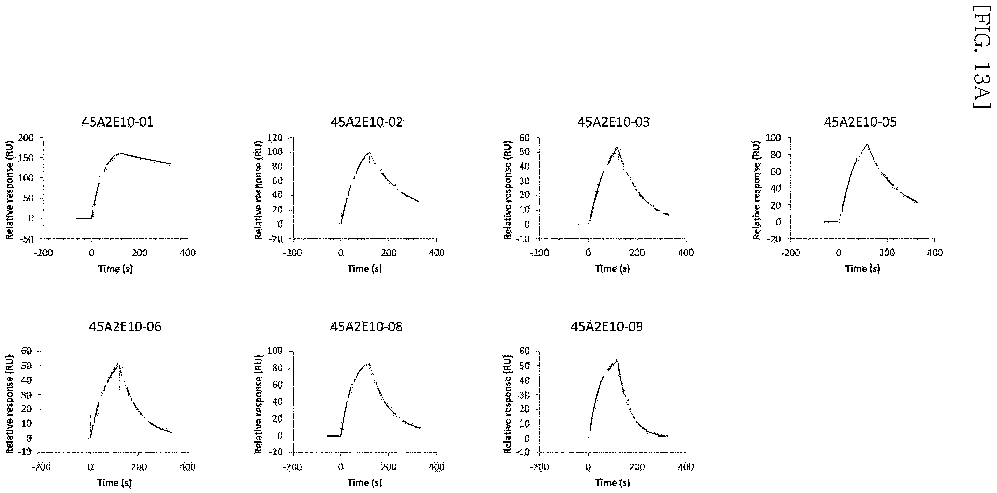

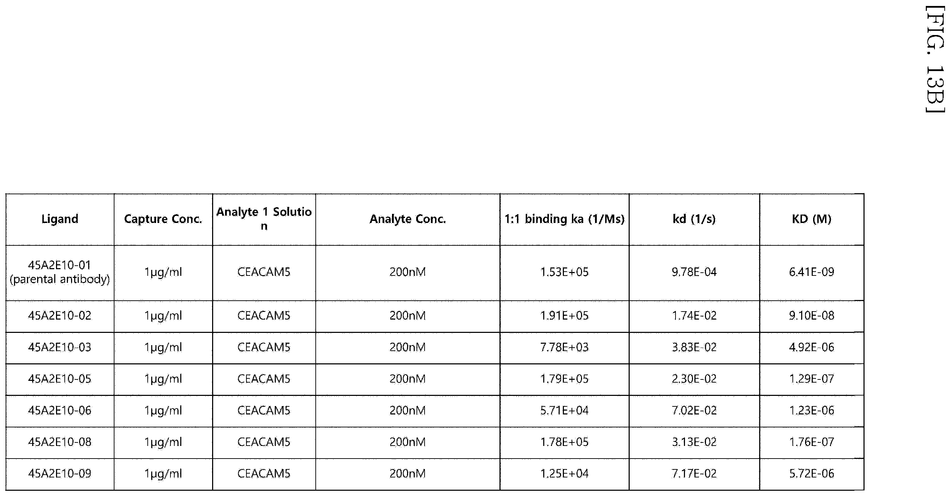

Hereinafter, the binding capacity of an antibody 45A2E10 produced by performing a humanization process according to an exemplary embodiment of the present disclosure will be described in detail with reference to FIGS. 13A and 13B.

FIGS. 13A and 13B are diagrams illustrating analysis of the binding affinity of an antibody 45A2E10 produced by performing a humanization process according to an exemplary embodiment of the present disclosure.

In order to analyze the binding affinity of various 45A2E10 clonal antibodies produced through the humanization process, first, 45A2E10 clones were humanized and produced, and among the clones, seven antibodies 45A2E10-02, 45A2E10-03, 45A2E10-05, 45A2E10-06, 45A2E10-08, and 45A2E10-09 showing binding affinity were selected. At this time, 45A2E10-01 corresponded to an antibody produced with a mouse sequence as a control, and the humanized antibodies of the clonal antibodies were produced by combining the same CDR 1 to 3 sequences and different framework sequences of the clonal antibodies, as shown in Tables 7 to 13 below.

**[Table 7]**

| CDR sequences of humanized 45A2E10 antibody | | | | |
|---|---|---|---|---|
| Antibody | Chain type | | sequence | Seq no. |
| humanized 45A2E10 antibody | VH | CDR 1 | NYGVH | 13 |
| | | CDR 2 | LIWAGGHTNYNSALMS | 14 |
| | | CDR 3 | EVRRDWYFDV | 15 |
| | VL | CDR 1 | RASENIYSYLT | 16 |
| | | CDR 2 | NAKILAE | 17 |
| | | CDR 3 | QHHFGTPFT | 18 |

**[Table 8]**

| Framework sequences of humanized 45A2E10-02 clonal antibody | | | |
|---|---|---|---|
| Antibody | Chain type | sequence | Seq no. |
| humanized 45A2E10-02 antibody | VH | QVQLQESGPGLVKPSETLSLTCTVSG GSISNYGVHWIRQPPGKGLEWIGLIW AGGHTNYNSALMSRVTISKDNSKSQI SLKLSSVTAADTAVYYCAREVRRDWY FDVWGQGTMVTVSS | 19 |
| | VL | DIQMTQSPSSLSASVGDRVTITCRAS ENIYSYLTWYQQKPGKAPKLLIYNAK ILAEGVPSRFSGSGSGTDFTLTISSL QPEDFATYYCQHHFGTPFTFGQGTKL EIK | 20 |

**[Table 9]**

| Framework sequences of humanized 45A2E10-03 clonal antibody | | | |
|---|---|---|---|
| Antibody | Chain type | sequence | Seq no. |
| humanized 45A2E10-03 antibody | VH | QVQLQESGPGLVKPSQTLSLTCAVYGGSF SNYGVHWIRQPPGKGLEWIGLIWAGGHTN YNSALMSRVTISKDNSKSQISLKLSSVTA ADTAVYYCAREVRRDWYFDVWGQGTMVTV SS | 21 |
| | VL | DIQMTQSPSSLSASVGDRVTITCRASENI YSYLTWYQQKPGKAPKLLIYNAKILAEGV PSRFSGSGSGTDFTLTISSLQPEDFATYY CQHHFGTPFTFGQGTKLEIK | 20 |

**[Table 10]**

| Framework sequences of humanized 45A2E10-05 clonal antibody | | | |
|---|---|---|---|
| Antibody | Chain type | sequence | Seq no. |
| humanized 45A2E10-05 | VH | QVQLQESGPGLVKPSETLSLTCTVSGGSI SNYGVHWIRQPPGKGLEWIGLIWAGGHTN YNSALMSRVTISKDNSKSQISLKLSSVTA | 19 |
| antibody | | ADTAVYYCAREVRRDWYFDVWGQGTMVTV SS | |
| | VL | DIQMTQSPSTLSASVGDRVTITCRASENI YSYLTWYQQKPGKAPKLLIYNAKILAEGV PSRFSGSGSGTEFTLTISSLQPDDFATYY CQHHFGTPFTFGQGTKLEIK | 22 |

**[Table 11]**

| Framework sequences of humanized 45A2E10-06 clonal antibody | | | |
|---|---|---|---|
| Antibody | Chain type | sequence | Seq no. |
| humanized 45A2E10-06 antibody | VH | QVQLQESGPGLVKPSQTLSLTCAVY GGSFSNYGVHWIRQPPGKGLEWIGL IWAGGHTNYNSALMSRVTISKDNSK SQISLKLSSVTAADTAVYYCAREVR RDWYFDVWGQGTMVTVSS | 21 |
| | VL | DIQMTQSPSTLSASVGDRVTITCRA SENIYSYLTWYQQKPGKAPKLLIYN AKILAEGVPSRFSGSGSGTEFTLTI SSLQPDDFATYYCQHHFGTPFTFGQ GTKLEIK | 22 |

**[Table 12]**

| Framework sequences of humanized 45A2E10-08 clonal antibody | | | |
|---|---|---|---|
| Antibody | Chain type | sequence | Seq no. |
| humanized 45A2E10-08 antibody | VH | QVQLQESGP GLVKP SETLSLTCTVSGG SISNYGVHWIRQPPGKGLEWIGLIWAG GHTNYNSALMSRVTISKDNSKSQISLK LSSVTAADTAVYYCAREVRRDWYFDVW GQGTMVTVSS | 19 |
| | VL | EIVMTQSPATLSVSPGERATLSCRASE NIYSYLTWYQQKPGQAPRLLIYNAKIL AEGIPARFSGSGSGTEFTLTISSLQSE DFAVYYCQHHFGTPFTFGQGTKLEIK | 23 |

**[Table 13]**

| Framework sequences of humanized 45A2E10-09 clonal antibody | | | |
|---|---|---|---|
| Antibody | Chain type | sequence | Seq no. |
| humanized 45A2E10-09 antibody | VH | QVQLQESGPGLVKPSQTLSLTCAVYG GSFSNYGVHWIRQPPGKGLEWIGLIW AGGHTNYNSALMSRVTISKDNSKSQI SLKLSSVTAADTAVYYCAREVRRDWY FDVWGQGTMVTVSS | 21 |
| | VL | EIVMTQSPATLSVSPGERATLSCRAS ENIYSYLTWYQQKPGQAPRLLIYNAK ILAEGIPARFSGSGSGTEFTLTISSL QSEDFAVYYCQHHFGTPFTFGQGTKL EIK | 23 |

The binding affinity analysis of the humanized antibodies was performed using Biacore 8K equipment. First, the antibodies were bound to a protein A chip at a concentration of 1 µg/ml, and then reacted with a CEACAM5 recombinant protein at a concentration of 200 nM to analyze the binding affinity of the antibodies above, which was illustrated in FIGS. 13A and 13B.

### 3-3. Confirmation of binding capacity of antibody 45A2E10 produced by performing humanization process

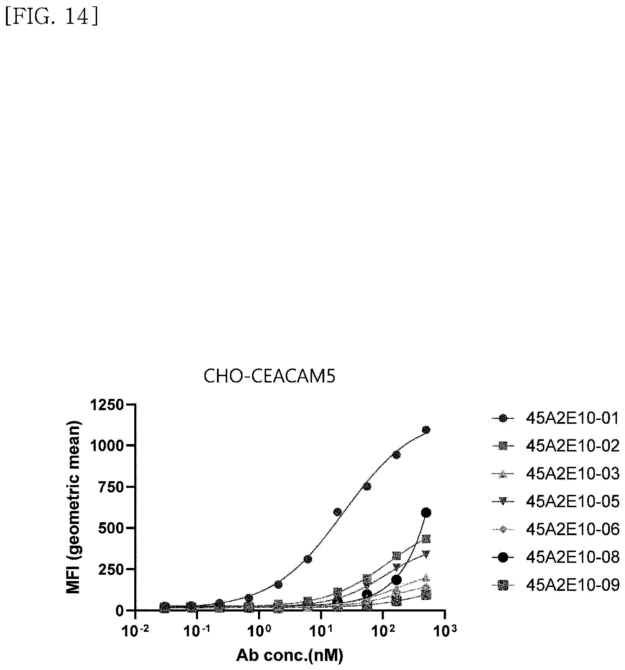

Hereinafter, the binding capacity of an antibody 45A2E10 produced by performing a humanization process according to an exemplary embodiment of the present disclosure to a CHO-k1 (CHO-CEACAM5) cell line expressing CEACAM5 will be described in detail with reference to FIG. 14.

FIG. 14 is a diagram illustrating the binding capacity of an antibody 45A2E10 produced by performing a humanization process according to an exemplary embodiment of the present disclosure to a CHO-k1 (CHO-CEACAM5) cell line expressing CEACAM5.

In order to confirm the binding capacity of the antibody 45A2E10 produced by the humanization process according to an exemplary embodiment of the present disclosure to the CHO-k1 (CHO-CEACAM5) cell line expressing CEACAM5, the CHO-k1 (CHO-CEACAM5) cell line expressing CEACAM5 was first dispensed into a 96-well plate at 3 × 10⁶ cells/well. Thereafter, humanized mAbs at various concentrations (0.025 nM to 500 nM) were diluted with a DPBS solution, dispensed into a CHO-CEACAM5 cell plate at each 50 µL/well, and then incubated at 4°C for 20 minutes. The plate was washed twice with 250 µL/well of Cold DPBS. (centrifugation at 1500 RPM and 4°C for 3 minutes) Then, the cells were dispensed with 50 µL/well of a solution in which a goat anti-human IgG Fcγ secondary antibody (Jackson ImmunoResearch) conjugated with Alexa Flour 647 was diluted with DPBS to a concentration of 15 µg/mL, and then the plate was wrapped with foil to block light and incubated at 4°C for 20 minutes, and washed twice with 250 µL/well of Cold DPBS. (centrifugation at 1500 RPM and 4°C for 3 minutes) Finally, 250 µL/well of Cold DPBS was dispensed, and the fluorescence was measured using a flow cytometer (MACSQuant^{®} Analyzer 10, Miltenyi Biotec) and the MFI (geometric mean) was analyzed using BD Flowjo.

As a result, referring to FIG. 14, it was confirmed that among the six antibodies produced by performing the humanization process according to an exemplary embodiment of the present disclosure, the 45A2E10-08 antibody showed particularly high binding capacity.

### 3-4. Confirmation of binding capacity of antibody 94E12E2 produced by performing humanization process

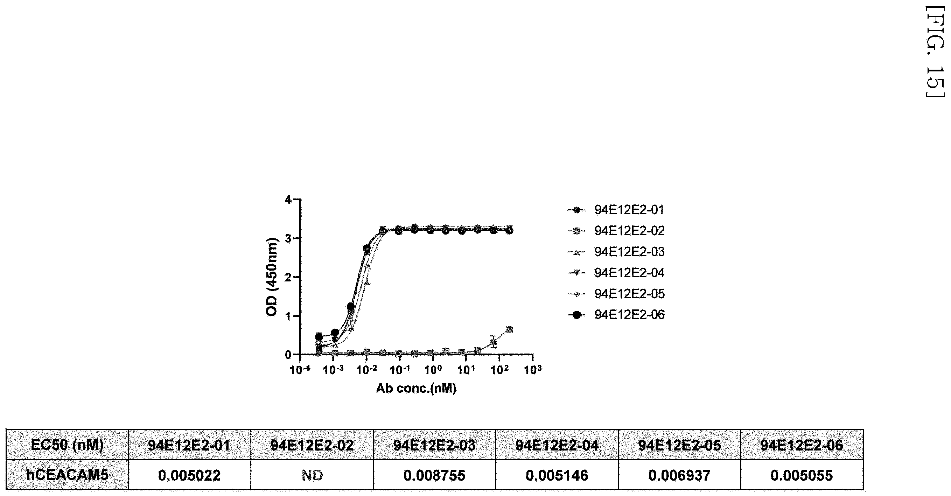

Hereinafter, the binding capacity of various clonal antibodies 94E12E2 produced by performing the humanization process according to an exemplary embodiment of the present disclosure will be described in detail through FIG. 15.

FIG. 15 is a diagram illustrating binding affinity of various clonal antibodies 94E12E2 produced by performing a humanization process according to an exemplary embodiment of the present disclosure confirmed by ELISA analysis.

First, the humanization process of the 94E12E2 clones was performed to produce five types of humanized antibodies 94E12E2-02, 94E12E2-03, 94E12E2-04, 94E12E2-05, and 94E12E2-06, and the binding affinity to the CEACAM5 recombinant protein was confirmed. At this time, 94E12E2-01 corresponds to an antibody produced with a mouse sequence as a control.

**[Table 14]**

| CDR sequences of humanized 94E12E2 antibody | | | | |
|---|---|---|---|---|
| Antibody | Chain type | | sequence | Seq no. |
| humanized 94E12E2 antibody | VH | CDR 1 | DNYMH | 24 |
| | | CDR 2 | WIDPENGDTEYDPKFQG | 25 |
| | | CDR 3 | ITMATPYPMDY | 26 |
| | VL | CDR 1 | SASTSVIYMH | 27 |
| | | CDR 2 | STSNLAS | 28 |
| | | CDR 3 | QQRSSYPLT | 29 |

**[Table 15]**

| Framework sequences of humanized 94E12E2-02 clonal antibody | | | |
|---|---|---|---|
| Antibody | Chain type | sequence | Seq no. |
| humanized 94E12E2-02 antibody | VH | EVQLVESGGGLVQPGGSLRLSCA ASGFNIKDNYMHWVRQAPGKGLE WVAWIDPENGDTEYDPKFQGRFT ISADTSKNTAYLQMNSLRAEDTA VYYCSRITMATPYPMDYWGQGTL VTVSS | 30 |
| | VL | DIQMTQSPSSLSASVGDRVTITC SASTSVIYMHWYQQKPGKAPKLL IYSTSNLASGVPSRFSGSRSGTD FTLTISSLQPEDFATYYCQQRSS YPLTFGQGTKVEIKR | 31 |

**[Table 16]**

| Framework sequences of humanized 94E12E2-03 clonal antibody | | | |
|---|---|---|---|
| Antibody | Chain type | sequence | Seq no. |
| humanized 94E12E2-03 antibody | VH | EVQLVESGGGLVQPGGSLRLSCAA SGFNIKDNYMHWVRQAPGKGLEWV AWIDPENGDTEYDPKFQGRATMSA DTSKNTAYLQMNSLRAEDTAVYYC NIITMATPYPMDYWGQGTLVTVSS | 32 |
| | VL | DIQMTQSPSSLSASVGDRVTITCS ASTSVIYMHWYQQKPGKAPKLLIY STSNLASGVPSRFSGSGSGTDYTL TISSLQPEDFATYYCQQRSSYPLT FGQGTKVEIKR | 33 |

**[Table 17]**

| Framework sequences of humanized 94E12E2-04 clonal antibody | | | |
|---|---|---|---|
| Antibody | Chain type | sequence | Seq no. |
| humanized 94E12E2-04 antibody | VH | EVQLVESGGGLVQPGGSLRLSCAA SGFNIKDNYMHWVRQAPGKGLEWI GWIDPENGDTEYDPKFQGRATMSA DTSKNTAYLQMNSLRAEDTAVYYC NIITMATPYPMDYWGQGTLVTVSS | 34 |
| | VL | DIQLTQSPSSLSASVGDRVTITCS ASTSVIYMHWFQQKPGKAPKLWIY STSNLASGVPSRFSGSGSGTDYTL TISSLQPEDFATYYCQQRSSYPLT FGQGTKVEIKR | 35 |

**[Table 18]**

| Framework sequences of humanized 94E12E2-05 clonal antibody | | | |
|---|---|---|---|
| Antibody | Chain type | sequence | Seq no. |
| humanized 94E12E2-05 antibody | VH | EVQLVQSGAEVKKPGATVKISC KVSGFNIKDNYMHWVQQAPGKG LEWIGWIDPENGDTEYDPKFQG RATMTADTSTDTAYMELSSLRS EDTAVYYCNIITMATPYPMDYW GQGTLVTVSS | 36 |
| | VL | EIVLTQSPATLSLSPGERATLS CSASTSVIYMHWFQQKPGQAPR LWIYSTSNLASGIPARFSGSGS GTDYTLTISSLEPEDFAVYYCQ QRSSYPLTFGQGTKLEIKR | 37 |

**[Table 19]**

| Framework sequences of humanized 94E12E2-06 clonal antibody | | | |
|---|---|---|---|
| Antibody | Chain type | sequence | Seq no. |
| humanized 94E12E2-06 antibody | VH | QVQLVQSGAEVKKPGASVKVSCKA SGFNIKDNYMHWVRQAPGQGLEWI GWIDPENGDTEYDPKFQGRATMTA DTSISTAYMELSRLRSDDTAVYYC NIITMATPYPMDYWGQGTLVTVSS | 38 |
| | VL | EIVLTQSPDFQSVTPKEKVTITCS ASTSVIYMHWFQQKPDQSPKLWIY STSNLASGVPSRFSGSGSGTDYTL TINSLEAEDAATYYCQQRSSYPLT FGQGTKLEIKR | 39 |

Thereafter, in order to confirm the binding capacity of the humanized 94E12E2 antibody, first, 30 µl of a CEACAM5 protein (Acrobiosystems) was dispensed per well of a 384-well microplate (Corning) at a concentration of 0.5 µg/ml, and incubated at 4°C for 16 hours, and then washed four times with 100 µL/well of PBST (DPBS + 0.05% Tween 20, Teknova). Then, the microplate was added with 80 µL/well of 1X PBS buffer containing 3% FBS, incubated at room temperature for 2.5 hours, and then washed four times with 100 µL/well of PBST in the same manner as above. At this time, the antibodies were prepared by diluting 3-fold to a concentration of 200 nM to 0.0004 nM with DPBS (Bining buffer) containing 0.5% Fetal Bovin Serum (FBS, Cytiva). The antibodies prepared as such were added to a CEACAM5-coated plate at 30 µL/well and incubated at room temperature for 1 hour. Thereafter, the plate was washed, and HRP (horseradish peroxidase) anti-mouse IgG Fcγ (secondary antibody, Jackson ImmunoResearch) was diluted to a concentration of 0.16 µg/mL (1 : 5000) in a binding buffer, added to the plate at 30 µL/well, and incubated at room temperature for 1 hour. Thereafter, the plate was washed in the same manner, added with a TMB (3,3',5,5'-tetramethylbenzidine, Invitrogen) substrate at 30 µL/well, and then incubated in the dark at room temperature for 10 minutes. Finally, the plate was added with 30 µL/well of a 0.16 M sulfuric acid (stop solution, Invitrogen) solution, and incubated in the dark for 10 minutes to stop the reaction, and the absorbance was measured at 450 nm (Synergy htx multimode reader, Biotek).

As a result, as illustrated in FIG. 15, it was confirmed that among the antibodies produced through the humanization process, the 94E12E2-04 and 94E12E2-06 antibodies showed particularly higher binding capacity.

### 3-5. Confirmation of binding capacity of antibody 94E12E2 produced by performing humanization process

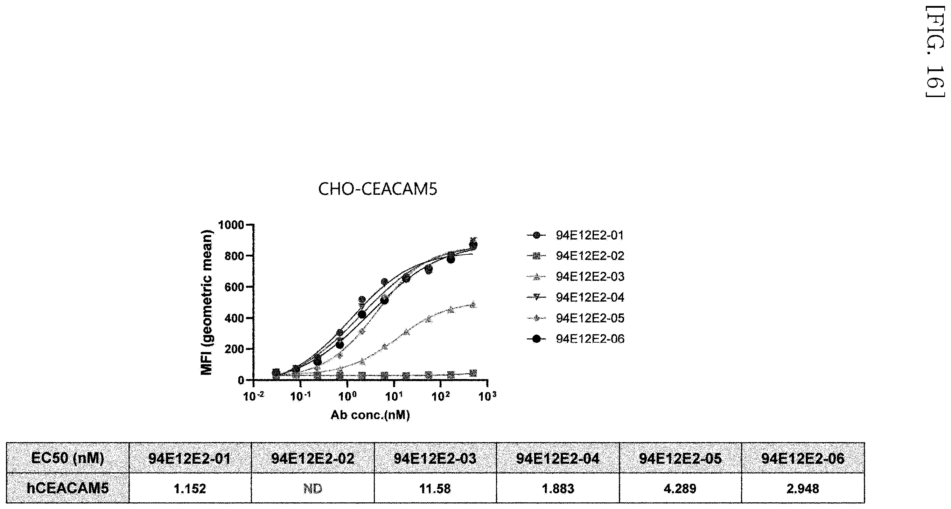

Hereinafter, the binding capacity of various clonal antibodies 94E12E2 produced by performing the humanization process according to an exemplary embodiment of the present disclosure will be described in detail through FIG. 16.

FIG. 16 is a diagram illustrating binding affinity of various clonal antibodies 94E12E2 produced by performing a humanization process according to an exemplary embodiment of the present disclosure confirmed by flow cytometry.

In order to confirm the binding capacity of the antibodies 94E12E2 produced by the humanization process according to an exemplary embodiment of the present disclosure to the CHO-k1 (CHO-CEACAM5) cell line expressing CEACAM5, first, the CHO-k1 (CHO-CEACAM5) cell line expressing CEACAM5 was dispensed into a 96-well plate at 3 × 10⁶ cells/well. Thereafter, humanized mAbs at various concentrations (0.025 nM to 500 nM) were diluted with a DPBS solution, dispensed into a CHO-CEACAMS cell plate at each 50 µL/well, and then incubated at 4°C for 20 minutes. The plate was washed twice with 250 µL/well of Cold DPBS. (centrifugation at 1500 RPM and 4°C for 3 minutes) Then, the cells were dispensed with 50 µL/well of a solution in which a goat anti-human IgG Fcγ secondary antibody (Jackson ImmunoResearch) conjugated with Alexa Flour 647 was diluted with DPBS to a concentration of 15 µg/mL, and then the plate was wrapped with foil to block light and incubated at 4°C for 20 minutes, and washed twice with 250 µL/well of Cold DPBS. (centrifugation at 1500 RPM and 4°C for 3 minutes) Finally, 250 µL/well of Cold DPBS was dispensed, and the fluorescence was measured using a flow cytometer (MACSQuant^{®} Analyzer 10, Miltenyi Biotec) and the MFI (geometric mean) was analyzed using BD Flowjo.

As a result, as illustrated in FIG. 16, it was confirmed that even among the antibodies produced through the humanization process, the 94E12E2-04 antibody showed particularly higher binding capacity.

### 4-1. Confirmation of binding capacity of antibody 36B5H8 clones with modified sequences corresponding to post-translational modification (PTM)

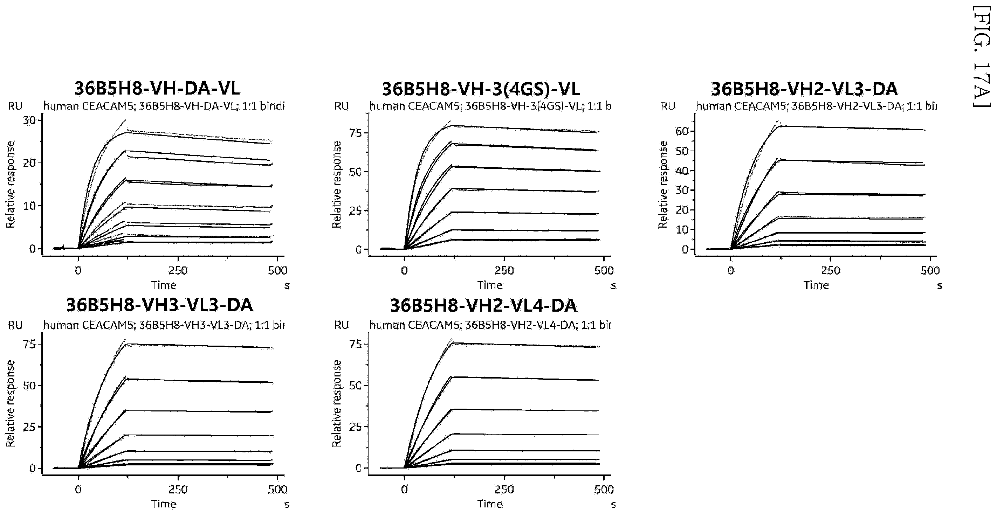

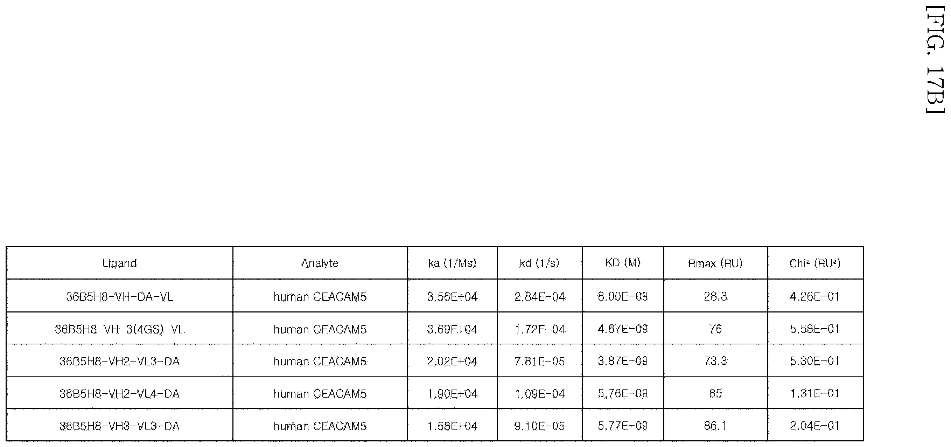

Hereinafter, the binding capacity of antibody 36B5H8 clones that have modified sequences corresponding to post-translational modification (PTM) according to an exemplary embodiment of the present disclosure will be described in detail through FIGS. 17A and 17B.

FIGS. 17A and 17B are diagrams illustrating analysis of binding affinity of antibody 36B5H8 clones that have modified sequences corresponding to post-translational modification (PTM) according to an exemplary embodiment of the present disclosure.

In order to analyze the binding affinity of 36B5H8 clonal antibodies with modified post-translational modification (PTM) portions, first, the antibodies were produced by modifying portions predicted to undergo post-translational modification in the selected humanized 36B5H8 clonal antibodies, and the binding affinity of the antibodies was analyzed using Biacore 8K equipment. First, the antibodies were bound to a CM5 chip, and then reacted with the CEACAM5 recombinant protein at various concentrations of 12.5 to 800 nM to analyze the binding affinity of these antibodies. At this time, 36B5H8-VH-3(4GS)-VL corresponds to an antibody prepared with a mouse sequence as a control, and 36B5H8-VH-DA-VL corresponds to an antibody prepared by modifying the post-translational modification portion of the mouse sequence as a control. The sequence of the humanized and post-translational modified (PTM) 36B5H8 antibody was prepared by having the same VH CDR1, VH CDR3, VL CDR1, VL CDR2, and VL CDR3 as the sequence of the humanized 36B5H8 antibody and a different portion of the amino acid sequence of VH CDR2 thereof.

**[Table 20]**

| CDR sequences of humanized 36B5H8 antibody | | | | |
|---|---|---|---|---|
| Antibody | Chain type | | sequence | Seq no. |
| humanized 36B5H8 antibody | VH | CDR 1 | GITFSRYAMS | 40 |
| | | CDR 2 | SISSDAITYYLD**S**VKG | 41 |
| | | CDR 3 | IYYYGSPFFDY | 42 |
| | VL | CDR 1 | KASQNVYTNVA | 43 |
| | | CDR 2 | SASNRYS | 44 |
| | | CDR 3 | QQYNNYPLFT | 45 |

**[Table 21]**

| Sequences of humanized and post-translational modified (PTM) 36B5H8 antibodies | | | | |
|---|---|---|---|---|
| Antibody | Chain type | | sequence | Seq no. |
| humanized 36B5H8 antibody | VH | CDR 1 | GITFSRYAMS | 40 |
| | | CDR 2 | SISSDAITYYLD**A**VKG | 46 |
| | | CDR 3 | IYYYGSPFFDY | 42 |
| | VL | CDR 1 | KASQNVYTNVA | 43 |
| | | CDR 2 | SASNRYS | 44 |
| | | CDR 3 | QQYNNYPLFT | 45 |

### 4-2. Confirmation of binding capacity of humanized and post-translational modified (PTM) antibodies

Hereinafter, the binding capacity of selected antibodies using CHO-K1-CEACAM5 cells after humanization and post-translational modification (PTM) according to an exemplary embodiment of the present disclosure will be described through FIG. 16.

FIG. 16 is a diagram illustrating binding capacity of selected antibodies using CHO-K1-CEACAM5 cells after humanization and post-translational modification (PTM) according to an exemplary embodiment of the present disclosure.

To confirm the binding of the selected antibodies using CHO-K1-CEACAM5 cells after humanization and post-translational modification (PTM), first, the CHO-K1-CEACAM5 cells were mixed with each antibody at a concentration of 0 to 45 µg/ml in 100 µl of 1X PBS and reacted at 4°C for 30 minutes. Then, the mixture was added with 2 ml of 1X PBS, centrifuged at 1500 rpm for 3 minutes to remove the supernatant, mixed with AF647-anti-human IgG, Fcγ fragment binding antibodies capable of confirming the first reacted antibodies at a concentration of 1 µg/ml in 100 µl of 1X PBS and then reacted with the cells at 4°C for 30 minutes. Finally, the cells were added with 2 ml of 1X PBS, centrifuged at 1500 rpm for 3 minutes to remove the supernatant, added with 300 µl of 1X PBS and measured using a flow cytometer.

As a result, as illustrated in FIG. 16, it was confirmed that all antibodies subjected to humanization and post-translational modification (PTM) as in the present disclosure showed higher binding capacity than the antibody 36B5H8-VH-DA-VL, which was prepared by modifying the post-translational modification portion of the mouse sequence as a control.

According to the results, the antibodies according to an exemplary embodiment of the present disclosure targeting CEACAM5 remaining on the cell surface specifically bound to CEACAM5 remaining on the cell surface without binding to soluble CEACAM5 to have excellent tumor cell recognition ability in a tumor microenvironment, thereby improving anticancer effects.

Although the exemplary embodiments of the present disclosure have been described in detail with reference to the accompanying drawings, the present disclosure is not limited thereto and may be embodied in many different forms without departing from the technical concept of the present disclosure. Therefore, the exemplary embodiments of the present disclosure are provided for illustrative purposes only but not intended to limit the technical concept of the present disclosure. The scope of the technical concept of the present disclosure is not limited thereto. Therefore, it should be appreciated that the aforementioned exemplary embodiments are illustrative in all aspects and are not restricted. The protective scope of the present disclosure should be construed on the basis of the appended claims, and all the technical ideas in the equivalent scope thereof should be construed as falling within the scope of the present disclosure.

## Claims

1. An anti-CEACAM5 antibody or antigen-binding fragment thereof specifically binding to CEACAM5 comprising:
(1) a heavy chain CDR1 including an amino acid sequence represented by SEQ ID NO: 1,
a heavy chain CDR2 including an amino acid sequence represented by SEQ ID NO: 2,
a heavy chain CDR3 including an amino acid sequence represented by SEQ ID NO: 3,
a light chain CDR1 including an amino acid sequence represented by SEQ ID NO: 4,
a light chain CDR2 including an amino acid sequence represented by SEQ ID NO: 5, and
a light chain CDR3 including an amino acid sequence represented by SEQ ID NO: 6;
(2) a heavy chain CDR1 including an amino acid sequence represented by SEQ ID NO: 13,
a heavy chain CDR2 including an amino acid sequence represented by SEQ ID NO: 14,
a heavy chain CDR3 including an amino acid sequence represented by SEQ ID NO: 15,
a light chain CDR1 including an amino acid sequence represented by SEQ ID NO: 16,
a light chain CDR2 including an amino acid sequence represented by SEQ ID NO: 17, and
a light chain CDR3 including an amino acid sequence represented by SEQ ID NO: 18;
(3) a heavy chain CDR1 including an amino acid sequence represented by SEQ ID NO: 24,
a heavy chain CDR2 including an amino acid sequence represented by SEQ ID NO: 25,
a heavy chain CDR3 including an amino acid sequence represented by SEQ ID NO: 26,
a light chain CDR1 including an amino acid sequence represented by SEQ ID NO: 27,
a light chain CDR2 including an amino acid sequence represented by SEQ ID NO: 28, and
a light chain CDR3 including an amino acid sequence represented by SEQ ID NO: 29; or
(4) a heavy chain CDR1 including an amino acid sequence represented by SEQ ID NO: 40,
a heavy chain CDR2 including an amino acid sequence represented by SEQ ID NO: 41 or 46,
a heavy chain CDR3 including an amino acid sequence represented by SEQ ID NO: 42,
a light chain CDR1 including an amino acid sequence represented by SEQ ID NO: 43,
a light chain CDR2 including an amino acid sequence represented by SEQ ID NO: 44, and
a light chain CDR3 including an amino acid sequence represented by SEQ ID NO: 45.

2. The anti-CEACAM5 antibody or antigen-binding fragment thereof of claim 1, wherein the anti-CEACAM5 antibody or antigen-binding fragment thereof specifically binds to CEACAM5 present on the cell surface without substantially binding to soluble CEACAM5 (sCEACAM5) that is not bound to the cell surface.

3. The anti-CEACAM5 antibody or antigen-binding fragment thereof of claim 1 or 2, wherein the anti-CEACAM5 antibody or antigen-binding fragment thereof is a humanized antibody.

4. An antibody-drug conjugate comprising the anti-CEACAM5 antibody or binding fragment thereof of claim 1 or 2 as an active ingredient.

5. A pharmaceutical composition for preventing or treating cancer, comprising the anti-CEACAM5 antibody or binding fragment thereof of any one of claim 1 or 2 as an active ingredient.

6. The pharmaceutical composition for preventing or treating cancer of claim 5, wherein the cancer is solid cancer.

7. The pharmaceutical composition for preventing or treating cancer of claim 6, wherein the solid cancer is any one of colon cancer, pancreatic cancer, lung cancer, stomach cancer, hepatocellular carcinoma, breast cancer, and thyroid cancer.

8. The pharmaceutical composition for preventing or treating cancer of claim 5, wherein the anti-CEACAM5 antibody or binding fragment thereof is used as a cell therapeutic agent for adaptive cell therapy (ACT).

9. A composition for inhibiting the proliferation of CEACAM5-expressing tumor cells, comprising the anti-CEACAM5 antibody or binding fragment thereof of claim 1 or 2.

10. A nucleic acid encoding the anti-CEACAM5 antibody or binding fragment thereof of claim 1 or 2.

11. A recombinant expression vector comprising the nucleic acid of claim 10.

12. A host cell transformed with the recombinant expression vector of claim 11.

13. A method for preventing or treating cancer in a subject, comprising administering the pharmaceutical composition of claim 5 to the subject.
